(19) Europäisches Patentamt
European Patent Office
Office européen des brevets

(11) Veröffentlichungsnummer: **0 200 024 B1**

(12) **EUROPÄISCHE PATENTSCHRIFT**

(45) Veröffentlichungstag der Patentschrift: **01.07.92**

(51) Int. Cl.$^5$: **C07D 213/85**, C07D 407/14, A61K 31/44

(21) Anmeldenummer: **86104594.6**

(22) Anmeldetag: **04.04.86**

Die Akte enthält technische Angaben, die nach dem Eingang der Anmeldung eingereicht wurden und die nicht in dieser Patentschrift enthalten sind.

(54) **3-Cyan-pyridine, Verfahren zu ihrer Herstellung und ihre pharmazeutische Verwendung.**

(30) Priorität: **30.04.85 DD 275789**
**23.05.85 DD 276639**
**23.05.85 DD 276640**
**23.05.85 DD 276641**
**23.05.85 DD 276642**
**30.12.85 DD 285598**
**14.02.86 DD 287057**
**14.02.86 DD 287058**
**14.02.86 DD 287059**

(43) Veröffentlichungstag der Anmeldung:
**05.11.86 Patentblatt 86/45**

(45) Bekanntmachung des Hinweises auf die Patenterteilung:
**01.07.92 Patentblatt 92/27**

(84) Benannte Vertragsstaaten:
**AT BE CH DE FR GB IT LI NL SE**

(56) Entgegenhaltungen:
**EP-A- 0 109 027**
**DE-A- 3 112 251**
**US-A- 3 660 415**
**US-A- 3 717 645**
**US-A- 4 362 734**

(73) Patentinhaber: **ARZNEIMITTELWERK DRESDEN GmbH**
**Wilhelm-Pieck-Strasse 35**
**O-8122 Radebeul(DE)**

(72) Erfinder: **Hagen, Volker, Dr.**
**Heinrich-Heine-Strasse 2**
**O-1020 Berlin(DE)**
Erfinder: **Hagen, Angela, Dr.**
**Heinrich-Heine-Strasse 2**
**O-1020 Berlin(DE)**
Erfinder: **Klauschenz, Erhard, Dr.**
**Wriezener Strasse 17**
**O-1125 Berlin(DE)**
Erfinder: **Heer, Sabine, Dipl.-Biol.**
**Trachenberger Strasse 25**
**O-8023 Dresden(DE)**
Erfinder: **Niedrich, Hartmut, Prof. Dr.**
**Theodor-Brugsch-Strasse 38**
**O-1115 Berlin(DE)**
Erfinder: **Lohmann, Dieter, Dr.**
**Hoflössnitzstrasse 30**
**O-8122 Radebeul(DE)**

**EP 0 200 024 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Erfinder: **Rumler, Andrea, Dipl.-Chem.**
**Tiroler Strasse 51**
**O-1100 Berlin(DE)**
Erfinder: **Heidrich, Hans-Joachim, Dipl.-Chem.**
**Ebereschenstrasse 32**
**O-8038 Dresden(DE)**
Erfinder: **Faust, Gottfried, Dr.**
**Ernst-Thälmann-Strasse 53**
**O-8122 Radebeul(DE)**
Erfinder: **Jänsch, Hans-Joachim, Dr.**
**Wilhelm-Pieck-Strasse 292**
**O-8122 Radebeul(DE)**
Erfinder: **Poppe, Hildegard, Dr.**
**Kieler Strasse 6**
**O-8080 Dresden(DE)**
Erfinder: **Femmer, Klaus, Dr.**
**Augustusweg 57**
**O-8122 Radebeul(DE)**
Erfinder: **Just, Elisabeth, Dipl.-Biol.**
**Weisbachstrasse 1**
**O-1034 Berlin(DE)**
Erfinder: **Hennig, Rosemarie, Vet.-Ing.**
**Althansweg 21**
**O-1140 Berlin(DE)**
Erfinder: **Muschik, Peter, Dipl.-Pharm.**
**Rummelsburger Strasse 593**
**O-1136 Berlin(DE)**
Erfinder: **Bürger, Sabine**
**Wilhelm-Guddorf-Strasse 24**
**O-1130 Berlin(DE)**

(74) Vertreter: **Puchberger, Rolf, Dipl. Ing. et al**
**Patentanwälte Dipl. Ing. Rolf Puchberger**
**Dipl. Ing. Georg Puchberger Singerstrasse 13**
**A-1010 Wien(AT)**

## Beschreibung

Die Erfindung betrifft neue 3-Cyan-pyridine der Formel I, in der $R^1$ Hydroxyethylamino. Bis-(2-hydroxyethyl)-amino, Morpholino, 2- oder 3-Hydroxy-propylamino, 1-Hydroxy-methyl-propylamino, 4-Hydroxy-butylamino, 2-Methoxy-ethylamino, 2,2-Dimethoxy-ethylamino, 2-(2-Hydroxy-ethoxy)-ethylamino, 2-Amino-ethylamino, 3-Diethylamino-propylamino, 3-Morpholino-propylamino,Piperazino, 4-Methyl-piperazino, 4-(2-Hydroxyethyl)-piperazino, 2-Acetylamino-ethylamino, 4-Acetyl-piperazino, 4-Propionyl-piperazino, 2-Nicotinoylamino-ethylamino, 2-Methoxy-ethoxy, 2-Hydroxy-ethoxy, 2- oder 3-Hydroxy-propoxy, 2,3-Dihydroxy-propoxy, 2-(2-Hydroxy-ethoxy)-ethoxy, 2-Acetoxy-ethylamino, 3-Acetoxy-propylamino, 2-Acetoxy-ethoxy, 3-Acetoxy-propoxy, Bis-(2-acetoxy-ethyl)-amino, 2,3-Bisacetoxy-propoxy, 2-Propionyloxy-ethylamino, 3-Propionyloxy-propylamino, 3-Propionyloxy-propoxy, 2-Nicotinoyloxy-ethylamino, 3-Nicotinoyloxy-propylamino oder 3-Nicotinoyloxy-propoxy bedeutet, $R^2$ für Pyrid-4-yl und $R^3$ für H oder Methyl stehen und ihre physiologisch verträglichen Säureadditionssalze.

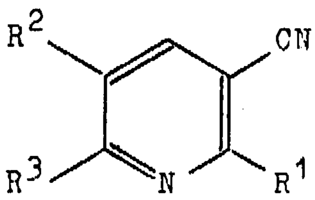

I

6-Unsubstituierte sowie 6-alkylsubstituierte 3-Cyan-5-(pyrid-4-yl)-1,2-dihydro-pyrid-2-one und ihre kardiotonische Wirkung sind beschrieben (s. z.B. G. Y. Lesher et al., US-PS 4 004 012. US-PS 4 199 586 und US-PS 4 264 609; G. Y. Lesher et al, US-PS 4 313 951, B. Singh, US-PS 4 347 363, US-PS 4 413 127. EP-PS 75 116; K. O. Gelotte, US. PS. 4 417 054).

Beschrieben worden sind weiter 6-unsubstituierte und 6-alkylsubstituierte 3-Cyan-2-ethylamino- bzw. 3-Cyan-2-methylamino-5-pyridyl-pyridine (G. Y. Lesher et al., US -PS 4 362 734) sowie 2-Methoxy- und 2-Ethoxy-3-cyan-5-pyridyl-pyridine (P. Nantka-Namirski et al.; J. Pharmacol. Pharm.. 30 (1978), 707, G. Y. Lesher et al.; US-PS 4 463 008). Ebenfalls bekannt ist 2-Amino-3-cyan-6-methyl-5-phenyl-pyridin (J. L. Miesel, US-PS 4 405 552, EP-PS 60 071).

Beschrieben sind auch 3-Amino- (bzw. 3-Nitro- bzw. 3-Carbamido)-2-(heteraalkylamino)-5-(pyrid-4-yl)-pyridine (G. Y. Lesher et al., US-PS 4 297 360, US-PS 4 374 141, GB-PS 2 073 185, DE-PS 3 112 251).

Bekannt sind das 6-unsubstituierte und das 6-methylsubstituierte 2-Chlor-3-cyan-5-(pyrid-4-yl)-pyridin (s. z.B. G. Y. Lesher et al., US-PS 4 264 603).

3-Cyan-2-oxaalkylamino-, 3-Cyan-2-aminoalkylamino- und 3-Cyan-2-oxaalkyloxy-5-(pyrid-4-yl)-pyridine sind bisher nicht bekannt.

Die kardiotonische Wirkung einer Reihe der aufgeführten Stoffe ist bekannt. Hinweise über bronchodilatatorische Eigenschaften gibt es bei 2-Amino- und 2-Alkylamino-3-amino-5-(pyrid-4-yl)-pyridinen. Antiarrhythmische und vasodilatatorische Wirkungen sind bei den genannten 2-Alkylamino- bzw 2-Alkyloxy-5-(pyrid-4-yl)-pyridinen nicht beschrieben worden.

Biologisch wirksame Heterocyclen werden häufig auch in Form ihrer physiologisch verträglichen Säureadditionssalze, wie Hydrochloride, Sulfate, Acetate, Fumarate oder Tartrate verabreicht (DE-OS 2 637 600).

Der Erfindung liegt die Aufgage zugrunde, neue biologisch aktive 3-Cyan-pyridine und Verfahren zu ihrer Herstellung sowie zu ihrer Anwendung zur Verfügung zu stellen.

Erfindungsgemäß werden Verbindungen der Formel I, worin die Reste $R^1$, $R^2$ und $R^3$ die oben genannte Bedeutung besitzen, hergestellt, indem man 3-Cyan-2-halogen-pyridine der Formel II, in der die Reste $R^2$ und $R^3$ die oben genannte Bedeutung besitzen,

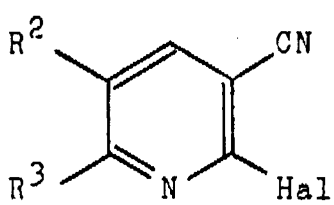

II

entweder

a) mit einer Verbindung der Formel

$R^1$ - H III

wenn diese ein Hydroxyalkylamin, Alkoxyalkylamin, Morpholin, Aminoalkylamin, Piperazin oder ein substituiertes Piperazin darstellt, in der $R^1$ die oben genannten Bedeutungen besitzt, in organischen Lösungsmitteln, wie Alkoholen, tertiären Aminen, Pyridin, Methylglykol, Dioxan, Dimethylformamid, Dimethylsulfoxid, Acetonitril, Gemischen dieser Lösungsmittel sowie den Heteraalkylaminen selbst, zu den 2-Oxaalkylamino- bzw, 2-Aminoalkylamino-Verbindungen oder

b) mit einer Verbindung der Formel

$R^1$ - H III

wenn diese ein Di- oder Tri-hydroxyalkan bzw. ein Alkoxyalkanol darstellt, in der $R^1$ die oben genannte Bedeutung besitzt, in Gegenwart von Basen, wie wasserfreie KOH oder NaOH, oder mit den entsprechenden Alkalimotallalkoholaten in dem jeweiligen Alkohol oder in inerten organischen Lösungsmitteln zu den 2-Oxaalkyloxy-Verbindungen oder

c) die 2-Hydroxyalkylamino- bzw. 2-Aminoalkylamino-Vorbindungen aus a) und die 2-Hydroxyalkyloxy-Verbindungen aus b) mit Säureanhydriden oder Säurehalogeniden, vorzugsweise in Gegenwart tertiärer Amine, zu den 2-Acyloxyalkylamino-, 2-Acylaminoalkylamino- bzw. 2-Acyloxyalkyloxy-Verbindungen

umsetzt. Die Reaktion nach c) wird vorteilhaft in Pyridin oder Trialkylaminen als Lösungsmittel vorgenommen.

Die neuen 3-Cyan-2-(heteraalkyl-1-hetera)-pyridine lassen sich mit anorganischen oder organischen Säuren in Wasser oder in organischen Lösungsmitteln in die entsprechenden Salze überführen.

Sowohl die freien Basen als auch ihre Salze sind biologisch aktiv. Insbesondere wurden kardiotone, antiarrhythmische, vasodilatatorische, bronchodilatatorische und antiallergische Wirkungen gefunden.

Kardiotonische Wirkungen wurden in der Reihe der 3-Cyan-2-oxaalkylamino-, 2-Aminoalkylamino-3-cyan- bzw. 3-Cyan-2-oxaalkyloxy-5-(pyrid-4-yl)-pyridine, der entsprechenden 6-methylsubstitutierten Verbindungen und den 5-Aryl-6-methyl-2-(heteraalkyl-1-hetera)-pyridinen der Formel I gefunden.

So zeigen beispielsweise 3-Cyan-2-(2-hydroxy-ethylamino)-, 3-Cyan-2-(3-hydroxy-propylamino) bzw. 3-Cyan-2-morpholino-5-(pyrid-4-yl)-pyridin sowie 3-Cyan-2-(2-hydroxy-ethoxy)-5-(pyrid-4-yl)-pyridin sowohl am Langendorff-Herzen des Meerschweinchens als auch am isolierten, spontanaktiven Vorhof des Meerschweinchens eine ausgeprägte und dosisabhängige positiv inotrope Wirkung bei relative geringer positiv chronotroper Aktivität. Eine gute positiv inotrope Wirkung bei gleichzeitig negativ chronotroper Wirkung wurde beispielsweise für 3-Cyan-2-[4-(furan-2-yl-carbonyl)-piperazino]-5-(pyrid-4-yl)-pyridin am isolierten, spontanaktiven Vorhofpräparat des Meerschweinchens gefunden.

Eine gute und dosisabhängige positiv inotrope Wirkung am isolierten, spontanaktiven Vorhofpräparat des Meerschweinchens wurde beispielsweise auch für 3-Cyan-2-(2-hydroxy-ethylamino)- bzw. 3-Cyan-2-(3-hydroxy-propylamino)-6-methyl-5-(pyrid-4-yl)-pyridin sowie 2-(2-Acetoxy-ethylamino)- und 2-(2-Acetoxy-ethoxy)-3-cyan-5-(pyrid-4-yl)-pyridin gefunden.

Die ausgeprägte und dosisabhängige positiv inotrope Wirkung von Verbindungen der Formel I wurde auch durch Untersuchungen am narkotisierten Minischwein und narkotisierten Hund nach i.v. Applikation nachgewiesen. So zeigen z.B. 3-Cyan-2-(2-hydroxy-ethylamino)- und 3-Cyan-2-morpholino-5-(pyrid-4-yl)-pyridin am narkotisierten Minischwein eine beträchtliche dosisabhängige Steigerung des Kontraktilitätsparameters $dp/dt_{max}$. Zusätzlich wurde eine dosisabhängige signifikante Senkung des diastolischen Blutdruckes gefunden.

Am narkotisierten Hund zeigen nach i.v. Applikation beispeilsweise 3-Cyan-6-methyl-2-morpholino-5-(pyrid-4-yl)-pyridin, 3-Cyan-6-methyl-2-morpholino-5-phenyl-pyridin, 3-Cyan-5-(3,4-dimethoxy-phenyl)-6-methyl-2-morpholino-pyridin, 2-(2-Acetoxy-ethylamino)-3-cyan-5-(pyrid-4-yl)-pyridin, 2-(2-Acetoxy-ethoxy)-3-cyan-5-(pyrid-4-yl)-pyridin und 2-(2-Propionyloxy-ethylamino)-3-cyan-5-(pyrid-4-yl)-pyridin dosisabhängige beträchtliche Steigerungen des Kontraktilitätsparameters $dp/dt_{max}$ sowie teilweise deutliche Senkungen des diastolischen Blutdruckes als relevantes Maß einer Abnahme des peripheren Gesamtwiderstandes.

Für beispielsweise 3-Cyan-2-(2-hydroxy-ethylamino)- und 3-Cyan-2-morpholino-5-(pyrid-4-yl)-pyridin wurde eine konzentrationsabhängige Hemmung der Noradrenalinkontraktion des Ductus deferens des Meerschweinchens gefunden, was ebenfalls auf eine periphere vasodilatatorische Wirkkomponente hindeutet.

Die bronchodilatatorische und antiallergische Wirkqualität von z.B. den 3-Cyan-2-heteraalkylamino-5-(pyrid-4-yl)-pyridinen wird beispielsweise durch die gute und dosisabhängige Schutzwirkung gegenüber der histamininduzierten Bronchokonstriktion am Meerschweinchen nach oraler Applikation belegt.
Die erfindungsgemäßen Verbindungen der Formel I lassen sich allein oder in Kombination untereinander oder mit weiteren kardiotonisch, antiarrhythmisch, vasodilatatorisch, bronchodilatatorisch und/oder antiallergisch wirksamen Verbindungen und/oder mit physiologisch verträglichen Trägern bzw. Verdünnungsmitteln verwenden.
Die Erfindung wird im folgenden anhand von Ausführungsbeispielen näher erläutert.

Ausführungsbeispiele:

Beispiel 1: 3-Cyan-2-(heteroalkylamino)-5-(pyrid-4-yl)-pyridine

Beispiel 1.1.

2-Brom-3-cyan-5-(pyrid-4-yl)-pyridin

19,7 g 3-Cyan-5-(pyrid-4-yl)-1,2-dihydro-pyrid-2-on, 120 ml $PBr_3$ und 2 ml Dimethylformamid werden 4 Stunden unter Rühren gekocht. Anschließend wird das überschüssige $PBr_3$ im Vakuum abdestilliert. Der Rückstand wird in Eiswasser gelöst, die Lösung mit 6 N $NH_3$-Lösung neutralisiert, das dabei anfallende Festprodukt abfiltriert und aus Isopropanol umkristallisiert.
Ausbeute: 11,5 g (44 % der Theorie). Schmp.: > 240 °C(Zers.).

Beispiel 1.2. bis 1.4

3-Cyan-2-(2-hydroxy-ethylamino)-5-(pyrid-4-yl)-pyridin

1,08 g 2-Chlor-3-cyan-5-(pyrid-4-yl)-pyridin werden mit Ethanolamin in geeigneten organischen Lösungsmitteln unter Rückfluß gekocht. Es wird stehengelassen, der ausfallende Feststoff abfiltriert, mit Wasser gewaschen und aus Isopropanol umkristallisiert.
Schmp.: 204-6 °C.

| Beispiel | Lösungsmittel | Reaktionszeit [h] | Ethanolamin [ml] | Ausbeute | |
|---|---|---|---|---|---|
| | | | | [g] | (%d.Th.) |
| 1.2 | 50 ml Ethanol | 5 | 2 | 0,84 | 70 |
| 1.3 | 5ml Pyridin | 3 | 0,75 | 1,00 | 83,3 |
| 1.4 | 20 ml Acetonitril | 1 | 2 | 0,78 | 64,9 |

Beispiel 1.5

3-Cyan-2-(2-hydroxy-ethylamino)-5-(pyrid-4-yl)-pyridin

1,3 g 2-Brom-3-cyan-5-(pyrid-4-yl)-pyridin werden mit 2 ml Aminoethanol in 50 ml Isopropanol 4 Stunden unter Rückfluß gekocht. Aufarbeitung erfolgt analog Beispiel 1.2. Ausbeute: 1,0 g (83,3 % der Theorie). Schmp.: 204-6 °C.

Beispiel 1.6

2-[Bis-(2-hydroxy-ethyl)-amino]-3-cyan-5-(pyrid-4-yl)-pyridin

6,45 g 2-Chlor-3-cyan-5-(pyrid-4-yl)-pyridin werden mit 18 ml Diethanolamin in 250 ml Ethanol/Methylglykol (4:1) 5 Stunden unter Rückfluß gekocht. Es wird über Nacht stehengelassen, eingeengt, mit Wasser versetzt, der ausfallende Feststoff abfiltriert und aus sek. Butanol umkristallisiert.
Ausbeute: 6,5 g (76 % der Theorie). Schmp. 184-8 °C (Zers.).

Beispiel 1.7

3-Cyan-2-morpholino-5-(pyrid-4-yl)-pyridin

6,45 g 2-Chlor-3-cyan-5-(pyrid-4-yl)-pyridin werden mit 10 ml Morpholin in 210 ml Isopropanol 4,5 Stunden unter Rückfluß gekocht. Es wird eingeengt, mit Wasser versetzt, der ausfallende Feststoff abgesaugt und aus Ethanol umkristallisiert.
Ausbeute: 6,39 g (80 % der Theorie). Schmp.: 126-8 °C.

Beispiel 1.8

3-Cyan-2-(3-hydroxy-propylamino)-5-(pyrid-4-yl)-pyridin

6,45 g 2-Chlor-3-cyan-5-(pyrid-4-yl)-pyridin werden mit 5,5 ml 3-Amino-propanol-(1) in 40 ml Pyridin 3 Stunden unter Rückfluß gekocht. Das Lösungsmittel wirk im Vakuum abdestilliert, der Rückstand mit Wasser versetzt, abgesaugt, aus Isopropanol und Wasser umkristallisiert und bei 110 °C getrocknet.
Ausbeute: 5,7 g (74,7 % der Theorie). Schmp.: 140-2 °C.

Beispiel 1.9

3-Cyan-2-(2-hydroxy-propylamino)-5-(pyrid-4-yl)-pyridin

2,16 g 2-Chlor-3-cyan-5-(pyrid-4-yl)-pyridin werden mit 2 ml 1-Amino-propanol-(2) in 90 ml Ethanol 5 Stunden unter Rückfluß gekocht. Es wird in der Wärme mit Aktivkohle versetzt, heiß filtriert, etwas eingeengt und in der Kälte stehengelassen. Der ausfallende Niederschlag wird abgesaugt.
Ausbeute: 1,93 g (76 % der Theorie). Schmp.: 177-9 °C.

Beispiel 1.10

3-Cyan-2-(1-hydroxymethyl-propylamino)-5-(pyrid-4-yl) pyridin

4,3 g 2-Chlor-3-cyan-5-(pyrid-4-yl)-pyridin werden mit 8 ml 2-Amino-butanol-(1) in 40 ml Pyridin 6 Stunden unter Rückfluß gekocht, Das Pyridin wird im Vakuum abrotiert, der Rückstand in 1 N HCl gelöst, mit Aktivkohle versetzt, filtriert und mit Ammoniak gefällt. Anschliessend wird aus Ethanol/Wasser umkristallisiert.
Ausbeute: 2,9 g (54,2 % der Theorie). Schmp.: 159-60 °C.

Beispiel 1.11

3-Cyan-2-morpholino-5-(pyrid-4-yl)-pyridin-fumarat

266 mg 3-Cyan-2-morpholino-5-(pyrid-4-yl)-pyridin werden in der Wärme in 10 ml EtOH gelöst und mit einer warmen Lösung von 116 mg Fumarsäure in 10 ml EtOH versetzt. Es wird stehengelassen und abgesaugt.
Ausbeute: 250 mg (77,1 % der Theorie).
$C_{15}H_{14}N_4O_1 \cdot 1/2C_4H_4O_4$ Schmp.: 195-9 °C.

Beispiel 1.12

3-Cyan-2-morpholino-5-(pyrid-4-yl)-pyridin-tartrat

266 mg 3-Cyan-2-morpholino-5-(pyrid-4-yl)-pyridin werden in der Wärme in 10 ml Ethanol gelöst und mit einer Lösung von 150 mg Weinsäure in Ethanol versetzt. Es wird eingeengt, stehengelassen und der ausfallende Niederschlag abgesaugt.
Ausbeute: 130 mg (38,1 % der Theorie).
$C_{15}H_{14}N_4O_1 \cdot 1/2C_4H_6O_6$
Schmp.: 153-6 °C

Beispiel 1.13

3-Cyan-2-morpholino-5-(pyrid-4-yl)-pyridin-hydrochloridmonohydrat

266 mg 3-Cyan-2-morpholino-5-(pyrid-4-yl)-pyridin werden in 1 ml 1 N HCl gelöst, mit 20 ml Ethanol versetzt und an der Luft stehengelassen. Die sich bildenden gelben Kristalle werden abgesaugt.
Ausbeute: 65 mg (20 % der Theorie).
$C_{15}H_{14}N_4O_1 \cdot HCl \cdot H_2O$ Schmp.: > 170 °C.

Beispiel 1.14

3-Cyan-2-(3-hydroxy-propylamino)-5-(pyrid-4-yl)-pyridinfumarat

254 mg 3-Cyan-2-(3-hydroxy-propylamino)-5-(pyrid-4-yl)-pyridin werden unter Erwärmen in 10 ml abs. Ethanol gelöst und mit 58 mg Fumarsäure in 10 ml Ethanol versetzt. Es wird bei 6-10 °C stehengelassen und abgesaugt.
Ausbeute: 220 mg (71 % der Theorie). Schmp.: 174-6 °C.

Beispiel 1.15

3-Cyan-2-(3-hydroxy-propylamino)-5-(pyrid-4-yl)-pyridintartrat

254 mg 3-Cyan-2-(3-hydroxy-propylamino)-5-(pyrid-4-yl)-pyridin werden unter Erwärmen in 10 ml abs. Ethanol gelöst und mit 75 mg Weinsäure in 5 ml Ethanol versetzt. Es wird bei 6-10 °C stehengelassen und abgesaugt.
Ausbeute: 180 mg (54,7 % der Theorie). Schmp.: 181-6 °C.

Beispiel 1.16

3-Cyan-2-(2-methoxy-ethylamino)-5-(pyrid-4-yl)-pyridin

Analog Beispiel 1.9 aus 2,16 g 2-Chlor-3-cyan-5-(pyrid-4-yl)-pyridin und 2 ml 1-Amino-2-methoxy-ethan in Ethanol.
Ausbeute: 1,53 g (62,4 % der Theorie). Schmp.: 141-2,5 °C.

Beispiel 1.17

3-Cyan-2-(4-hydroxy-butylamino)-5-(pyrid-4-yl)-pyridin

Analog Beispiel 1.9 aus 2,16 g 2-Chlor-3-cyan-5-(pyrid-4-yl)-pyridin und 2 ml 1-Amino-butanol-(4) und Umkristallisation aus Ethanol.
Ausbeute: 1,9 g (70,82 % der Theorie). Schmp.: 170-1 °C.

Beispiel 1.18

3-Cyan-2-(2,2-dimethoxy-ethylamino)-5-(pyrid-4-yl)-pyridin

2,16 g 2-Chlor-3-cyan-5-(pyrid-4-yl)-pyridin werden mit 3 ml 2,2-Dimethoxy-ethylamin in 80 ml Methanol 5 Stunden unter Rückfluß gekocht. Es wird in der Wärme mit Aktivkohle versetzt, heiß filtriert und über Nacht stehengelassen. Der ausfallende Niederschlag wird abgesaugt und aus Methanol umkristallisiert.
Ausbeute: 1,5 g (52,8 % der Theorie). Schmp.: 128-9 °C.

Beispiel 1.19

3-Cyan-2-[2-(2-hydroxy-ethoxy)-ethylamino]-5-(pyrid-4-yl)-pyridin

4,3 g 2-Chlor-3-Cyan-5-(pyrid-4-yl)-pyridin werden mit 5 ml 2-(2-Amino-ethoxy)-ethanol-(1) in 150 ml Ethanol 5 Stunden unter Rückfluß gekocht. Es wird mit Aktivkohle versetzt, heiß filtriert, stehengelassen und

abgesaugt. Durch Einengen der Mutterlauge, Absaugen und Waschen mit Ethanol und Ether wird weiteres Produkt erhalten.
Ausbeute: 3,87 g (85,1 % der Theorie). Schmp.: 191-2 °C.

Beispiel 1.20

3-Cyan-2-(3-morpholino-propylamino)-5-(pyrid-4-yl)-pyridin

1,08 g 2-Chlor-3-cyan-5-(pyrid-4-yl)-pyridin werden mit 3 g 3-Morpholino-propylamin in 50 ml Ethanol 5 Stunden unter Rückfluß gekocht. Es wird mit Wasser versetzt, abgesaugt und aus Isopropanol unter Zusatz von Aktivkohle umkristallisiert.
Ausbeute: 1,3 g (81 % der Theorie). Schmp.:164-6 °C.

Beispiel 1.21

3-Cyan-2-(3-diethylamino-propylamino)-5-(pyrid-4-yl)-pyridin

4,3 g 2-Chlor-3-cyan-5-(pyrid-4-yl)-pyridin werden mit 5 g 3-Diethylamino-propylamin in 200 ml i-Propanol 3 Stunden unter Rückfluß gekocht. Es wird mit Aktivkohle versetzt, filtriert, eingeengt, mit Wasser versetzt, mit $CHCl_3$ ausgeschüttelt, die $CHCl_3$-Phase über $MgSO_4$ getrocknet, eingeengt und unter Zusatz von Hexan gefällt. Die ausgefallenen Kristalle werden abgesaugt und aus i-Propanol umkristallisiert.
Ausbeute: 5,4 g (87 % der Theorie). Schmp.:95,5-6,5 °C.

Beispiel 1.22

3-Cyan-2-(1,3-dimethyl-3-dimethylamino-butylamino)-5-(pyrid-4-yl)-pyridin

2,16 g 2-Chlor-3-cyan-5-(pyrid-4-yl)-pyridin werden mit 2,5 g 2-Dimethylamino-4-amino-2-methyl-pentan in 100 ml Ethanol 5 Stunden unter Rückfluß gekocht. Es wird mit Aktivkohle versetzt, filtriert, eingeengt, mit Wasser versetzt und stehengelassen. Die ausgefallenen Kristalle werden abgesaugt und aus Ethanol umkristallisiert.
Ausbeute: 2,8 g (86,4 % der Theorie). Schmp.:116-9 °C.

Beispiel 1.23

3-Cyan-2-piperazino-5-(pyrid-4-yl)-pyridin

6,47 g 2-Chlor-3-cyan-5-(pyrid-4-yl)-pyridin werden mit 6,0 g Piperazin in 200 ml Ethanol 4 Stunden unter Rückfluß gekocht. Es wird auf ca. 40 °C abgekühlt, filtriert, die Lösung mit Aktivkohle versetzt, erneut filtriert, stark eingeengt und mit etwas Wasser und 2N $NH_3$ versetzt. Das nach Stehenlassen in der Kälte ausfallende Produkt wird abgesaugt und aus Wasser umkristallisiert. Anschließend wird das zunächst anfallende bei 44-48 °C schmelzende Hydrat durch Trocknen bei 110 °C im Vakuum in die wasserfreie Verbindung überführt.
Ausbeute: 3,9 g (49,0 der Theorie). Schmp.:120-3 °C.

Beispiel 1.24

3-Cyan-2-piperazino-5-(pyrid-4-yl)-pyridin

1,3 g 2-Brom-3-cyan-5-(pyrid-4-yl)-pyridin werden mit 1,5 g Piperazin in 50 ml Ethanol 4 Stunden unter Rückfluß gekocht. Aufarbeitung erfolgt analog Beispiel 1.23
Ausbeute: 1,52 g (57,3 % der Theorie). Schmp.: 120-3 °C.

Beispiel 1.25

3-Cyan-2-(4-methyl-piperazino)-5-(pyrid-4-yl)-pyridin

2,16 g 2-Chlor-3-cyan-5-(pyrid-4-yl)-pyridin werden mit 2,5 ml N-Methylpiperazin in 80 ml Ethanol 2,5

Stunden unter Rückfluß gekocht. Es wird mit Aktivkohle versetzt, filtriert, am Rotationsverdampfer stark eingeengt und mit wenig Wasser und etwas $NH_3$-Lösung versetzt. Das nach Stehenlassen in der Kälte anfallende bei 39-41 °C schmelzende Hydrat wird anschließend in Vakuum über $P_2O_5$ getrocknet.
Ausbeute: 1,67 g (59,9 % der Theorie). Schmp.:100-2 °C.

Beispiel 1.26

3-Cyan-2-[4-(2-hydroxy-ethyl)-piperazino]-5-(pyrid-4-yl)-pyridin

3,23 g 2-Chlor-3-cyan-5-(pyrid-4-yl)-pyridin werden mit 3 ml N-(2-Hydroxy-ethyl)-piperazin in 80 ml Ethanol 5 Stunden unter Rückfluß gekocht. Es wird mit Aktivkohle versetzt, filtriert, am Rotationsverdampfer stark eingeengt und mit etwas 1N $NH_3$-Lösung versetzt. Das nach Stehenlassen in der Kälte anfallende niedrigschmelzende Hydrat wird aus wenig Ethanol/Wasser unter Zusatz von Aktivkohle umkristallisiert. Anschließend wird über $P_2O_5$ im Vakuum getrocknet.
Ausbeute: 2,21 g (47,6 % der Theorie). Schmp.:125-7 °C.

Beispiel 1.27

3-Cyan-2-[4-(furan-2-yl-carbonyl)-piperazino]-5-(pyrid-4-yl)-pyridin

2,16 g 2-Chlor-3-cyan-5-(pyrid-4-yl)-pyridin werden mit 1,8 g N-(Furan-2-yl-carbonyl)-piperazin und 0,7 g $K_2CO_3$ in 70 ml Ethanol 2,5 Stunden unter Rückfluß gekocht. Es wird abgekühlt, mit Wasser versetzt, filtriert, der Niederschlag in verd. HCl aufgenommen, wieder mit verd. $NH_3$-Lösung gefällt, abgesaugt und aus Dimethylformamid unter Zusatz von Aktivkohle umkristallisiert.
Ausbeute: 1,98 g (55,1 % der Theroie). Schmp.:291-3 °C.

Beispiel 1.28

2-(2-Amino-ethylamino)-3-cyan-5-(pyrid-4-yl)-pyridin

2,16 g 2-Chlor-3-cyan-5-(pyrid-4-yl)-pyridin werden mit 3 ml Ethylendiamin in 80 ml Ethanol 3 Stunden unter Rückfluß gekocht. Es wird mit Aktivkohle versetzt, heiß filtriert, am Rotationsverdampfer eingeengt, mit etwas Wasser und $NH_3$-Lösung versetzt und in der Kälte stehen gelassen. Der ausfallende Niederschlag wird aus Ethanol/Wasser umkristallisiert.
Ausbeute: 1,3 g (54,4 % der Theorie). Schmp.:158-60 °C.

Beispiel 1.29

2-(2-Acetylamino-ethylamino)-3-cyan-5-(pyrid-4-yl)-pyridin

0,48 g 2-(2-Amino-ethylamino)-3-cyan-5-(pyrid-4-yl)-pyridin werden in 5 ml Pyridin unter leichtem Erwärmen gelöst, mit 0,3 ml Acetanhydrid versetzt und 0,5 Std. stehengelassen. Der ausfallende Feststoff wird abgesaugt und aus Acetonitril umkristallisiert.
Ausbeute: 0,3 g (53,3 % der Theorie). Schmp.:204-5,5 °C.

Beispiel 1.30

2-(4-Acetyl-piperazino)-3-cyan-5-(pyrid-4-yl)-pyridin

1,06 g 3-Cyan-2-piperazino-5-(pyrid-4-yl)-pyridin werden in 10 ml Pyridin mit 0,8 ml Acetanhydrid 5 Min. gerührt. Es wird abgesaugt und mit Ethanol und Ether gewaschen.
Ausbeute: 0,96 g (78,2 % der Theorie). Schmp.:233-4,5 °C.

Beispiel 1.31

3-Cyan-2-(4-propionyl-piperazino)-5-(pyrid-4-yl)-pyridin

Analog Beispiel 1.30 aus 1,06 g 3-Cyan-2-piperazino-5-(pyrid-4-yl)-pyridin und 1,2 ml Propionsäureanhydrid.
Ausbeute: 1,12 g (87,2 % der Theorie). Schmp.:214-6 °C.

Beispiel 1.32

2-(4-Benzoyl-piperazino)-3-cyan-5-(pyrid-4-yl)-pyridin

1,06 g 3-Cyan-2-piperazino-5-(pyrid-4-yl)-pyridin in 10 ml Pyridin werden mit 1,8 ml Benzoylchlorid 10 Min. gerührt. Es wird mit 10 ml Wasser versetzt, abgesaugt und mit Wasser, Ethanol und Ether gewaschen.
Ausbeute: 1,06 g (86,6 % der Theorie). Schmp.:208-9 °C.

Beispiel 1.33

3-Cyan-2-(2-nicotinoylamino-ethylamino)-5-(pyrid-4-yl)-pyridin-monohydrat

0,7 g Nicotinsäure in 2 ml Pyridin werden mit 0,3 ml $SO_2Cl_2$ versetzt, es wird auf 100 °C erwärmt, abgekühlt mit 0,96 g 2-(2-Amino-ethylamino)-3-cyan-5-(pyrid-4-yl)-pyridin versetzt, 10 Min. bei 100 °C gerührt, abgekühlt, eingeengt, mit Wasser versetzt, auf pH 8 gebracht, abgesaugt, und aus Ethanol, unter Zusatz von Aktivkohle, umkristallisiert.
Ausbeute: 0.21 g (29,0 % der Theorie). Schmp.:208,5-10 °C.

Beispiel 2: 3-Cyan-2-(oxaalkyloxy)-5-(pyrid-4-yl)-pyridine

Beispiel 2.1

3-Cyan-2-(2-methoxy-ethoxy)-5-(pyrid-4-yl)-pyridin

0,7 g Natrium werden mit 60 ml Methylglykol versetzt und nach dem Auflösen werden 6,47 g 2-Chlor-3-cyan-5-(pyrid-4-yl)-pyridin unter Rühren hinzugegeben. Es wird 0,5 Stunden unter Rückfluß gekocht, abgekühlt, mit Wasser versetzt, der ausfallende Feststoff abgesaugt und aus Ethanol unter Zusatz von Aktivkohle umkristallisiert.
Ausbeute: 6,1 g (79,6 % der Theorie). Schmp.: 160-1 °C.

Beispiel 2.2

3-Cyan-2-(2-hydroxy-ethoxy-)-5-(pyrid-4-yl)-pyridin

Zu einer Suspension von 2,24 g KOH in 150 ml Ethylenglykol werden 8,62 g 2-Chlor-3-cyan-5-(pyrid-4-yl)-pyridin unter Rühren gegeben. Es wird 0,5 Stunden bei 90 - 100 °Cgerührt, mit Aktivkohle versetzt, filtriert, abgekühlt, mit Wasser versetzt, der ausgefallene Feststoff abgesaugt und aus Methylglykol/Ethanol umkristallisiert.
Ausbeute: 8,2 g (85,0 % der Theorie). Schmp.: 185-9 °C.

Beispiel 2.3

3-Cyan-2-(3-hydroxy-propoxy)-5-(pyrid-4-yl)-pyridin

230 mg Natrium werden mit 30 ml Propandiol-(1,3) versetzt und nach dem Auflösen werden 2,15 g 2-Chlor-3-cyan-5-(pyrid-4-yl)-pyridin unter Rühren hinzugefügt. Es wird 1 Stunde bei 100 °C gerührt, abgekühlt, mit Wasser versetzt, der ausgefallene Feststoff abgesaugt und aus Ethanol umkristallisiert.
Ausbeute: 1,68 g (65,8 % der Theorie). Schmp.: 134-8 °C.

Beispiel 2.4.

3-Cyan-2-(2-hydroxy-propoxy)-5-(pyrid-4-yl)-pyridin

560 mg KOH werden unter Erwärmen in 30 ml Propandiol-(1,2) gelöst. Anschließend werden 2,15 g 2-Chlor-3-cyan-5-(pyrid-4-yl)-pyridin hinzugefügt, es wird 0.5 Stunden bei 140 °C gerührt, das überschüssige

Propandiol-(1,2) im Vakuum zur Wiedergewinnung abdestilliert, mit Wasser versetzt, abgesaugt und aus Methylglykol/Ethanol/Wasser unter Zusatz von Aktivkohle umkristallisiert.
Ausbeute: 1,9 g (74,4 % der Theorie). Schmp.: 203-5 °C.

Beispiel 2.5

3-Cyan-2-(2,3-dihydroxy-propoxy)-5-(pyrid-4-yl)-pyridin

560 mg KOH werden unter Erwärmen in 30 ml Glycerin gelöst, und anschließend werden 2,15 g 2-Chlor-3-cyan-5-(pyrid-4-yl)-pyridinhinzugefügt. Es wird o,5 Stunden bei 80 - 90 °C gerührt, die Suspension abgekühlt, mit Wasser versetzt, der Niederschlag abgesaugt, mit Acetonitril ausgezogen und aus Ethanol/Methylglykol unter Zusatz von Aktivkohle umkristallisiert.
Ausbeute: 1,7 g (62,7 % der Theorie). Schmp.: 207-11 °C.

Beispiel 2.6

3-Cyan-2-[2-(hydroxy-ethoxy)-ethoxy]-5-(pyrid-4-yl)-pyridin

560 mg KOH werden unter Erwärmen in 30 ml Diethylenglykol gelöst. Anschließend werden 2,15 g 2-Chlor-3-cyan-5-(pyrid-4-yl)-pyridin hinzugefügt. Es wird 0,5 Std. bei 100 °C gerührt, mit Aktivkohle versetzt, filtriert, abgekühlt, abgesaugt und aus Ethanol umkristallisiert.
Ausbeute: 1,3 g (45,6 % der Theorie). Schmp.: 122-4 °C.

Beispiel 3: 2-substituierte-3-Cyan-6-methyl-5-(pyrid-4-yl)-pyridine

Beispiel 3.1

2-Chlor-3-cyan-6-methyl-5-(pyrid-4-yl)-pyridin

4 g 3-Cyan-6-methyl-5-(pyrid-4-yl)-1,2-dihydro-pyrid-2-on werden mit 24 ml $POCl_3$ und 0,5 ml Dimethylformamid 6 Stunden unter Rückfluß gekocht. Nach Abkühlen auf Raumtemperatur wird die Reaktionslösung vorsichtig in 100 ml Eiswasser gegossen, mit 6N $NH_3$ neutralisiert, der ausfallende Feststoff abgesaugt und mit Wasser gewaschen. Der Niederschlag wird wieder in verdünnter HCl gelöst, die Lösung in der Wärme mit Aktivkohle versetzt, filtriert, mit $NH_3$ neutralisiert, der ausfallende Niederschlag abgesaugt, mit Wasser gewaschen und getrocknet.
Ausbeute: 3,8 g (87,5 % der Theorie). Schmp.: 155-7 °C.

Beispiel 3.2

3-Cyan-6-methyl-2-morpholino-5-(pyrid-4-yl)-pyridin

1 g 2-Chlor-3-cyan-6-methyl-5-(pyrid-4-yl)-pyridin werden mit 2 ml Morpholin und 1 ml Pyridin in 25 ml Acetonitril 5 Stunden unter Rückfluß gekocht. Nach Eingengen des Ansatzes auf die Hälfte und Stehen in der Kälte wird abgesaugt, das Produkt in verdünnter HCl gelöst, die Lösung mit Aktivkohle versetzt, filtriert und mit $NH_3$ neutralisiert. Der ausfallende Niederschlag wird abgesaugt und aus Ethanol/Wasser umkristallisiert.
Ausbeute: 0,7 g (57,2 % der Theorie). Schmp.: 169-71 °C.

Beispiel 3.3

3-Cyan-2-(2-hydroxy-ethylamino)-6-methyl-5-(pyrid-4-yl)-pyridin

1 g 2-Chlor-3-cyan-6-methyl-5-(pyrid-4-yl)-pyridin werden mit 2 ml 1-Amino-ethanol-(2) in 25 ml Ethanol 5 Stunden am Rückfluß erhitzt. Es wird in der Wärme mit Aktivkohle versetzt, heiß filtriert, eingeengt und in der Kälte stehengelassen. Der ausfallende Niederschlag wird abgesaugt.
Ausbeute: 0,7 g (62,9 % der Theorie). Schmp.: 192-4 °C.

Beispiel 3.4

3-Cyan-2-(3-hydroxy-propylamino)-6-methyl-5-(pyrid-4-yl)-pyridin

1 g 2-Chlor-3-cyan-6-methyl-5-(pyrid-4-yl)-pyridin werden mit 2 ml 1-Amino-propanol-(3) und 1 ml Pyridin in 25 ml Ethanol 5 Stunden am Rückfluß gekocht. Der Ansatz wird im Vakuum eingeengt, der Rückstand in 1N HCl gelöst, mit Aktivkohole versetzt, filtriert und mit $NH_3$ gefällt. Anschließend wird aus Ethanol/Wasser umkristallisiert und das Produkt bei 110 °C getrocknet.
Ausbeute: 0.65 g (55,4 % der Theorie). Schmp.: 122-4 °C.

Beispiel 3.5

3-Cyan-2-(2-hydroxy-propylamino)-6-methyl-5-(pyrid-4-yl)-pyridin

1 g 2 Chlor-3-cyan-6-methyl-5-(pyrid-4-yl)-pyridin werden mit 2 ml 1-Amino-propanol-(2) und 1 ml Pyridin in 35 ml Methylglykol 6 Stunden am Rückfluß gekocht. Es wird im Vakuum etwas eingeengt, mit Wasser versetzt und in der Kälte stehengelassen. Der ausfallende Niederschlag wird abgesaugt und aus iso-Propanol umkristallisiert.
Ausbeute: 0.8 g (68.2 % der Theorie). Schmp.: 178-81 °C.

Beispiel 3.6

2-[Bis-(2-hydroxyethyl)-amino]-3-cyan-6-methyl-5-(pyrid-4-yl)-pyridin

1 g 2-Chlor-3-cyan-6-methyl-5-(pyrid-4-yl)-pyridin werden mit 2 ml Diethanolamin und 1 ml Pyridin in 25 ml iso-Propanol 5 Stunden am Rückfluß gekocht. Das iso-Propanol wird im Vakuum abrotiert, der Rückstand in 1N HCl gelöst, mit Aktivkohle versetzt, filtriert und mit $NH_3$ gefällt. Anschließend wird aus Ethanol/Wasser umkristallisiert und bei 120 °C getrocknet.
Ausbeute: 0,85 g (65,2 % der Theorie). Schmp.: 133-36 °C.

Beispiel 3.7

3-Cyan-6-methyl-2-morpholino-5-(pyrid-4-yl)-pyridinfumarat

280 mg 3-Cyan-6-methyl-2-morpholino-5-(pyrid-4-yl)-pyridin werden unter Erwärmen in 20 ml Ethanol gelöst und mit 116 mg Fumarsäure in 10 ml Ethanol versetzt. Es wird bei 6-10 °C stehengelassen und abgesaugt.
Ausbeute: 260 mg (77 % der Theorie). Schmp.: 179-80 °C.

Beispiel 3.8

3-Cyan-2-(2-methoxy-ethoxy)-6-methyl-5-(pyrid-4-yl)-pyridin

Analog Beispiel 2.1 durch Auflösen von 0.15 g Natrium in 30 ml Methylglykol und 45 Min. Erhitzen mit 1,5 g 2-Chlor-3-cyan-6-methyl-5-(pyrid-4-yl)-pyridin bei 90-100 °C.
Ausbeute: 1,4 g (68,8 % der Theorie). Schmp.: 82,5-4 °C.

Beispiel 3.9

3-Cyan-2-(2-hydroxy-ethoxy)-6-methyl-5-(pyrid-4-yl)-pyridin

Analog Beispiel 2.2 aus 2,2 g KOH und 9,2 g 2-Chlor-3-cyan-6-methyl-5-(pyrid-4-yl)-pyridin durch Rühren bei 90-100 °C in 100 ml Ethylenglycol und Umkristallisation aus Ethanol/Chloroform unter Zusatz von Aktivkohle.
Ausbeute: 3,6 g (35,2 % der Theorie). Schmp.: 194-5 °C.

Beispiel 3.10

3-Cyan-2-(2,3-dihydroxy-propoxy)-6-methyl-5-(pyrid-4-yl)-pyridin

Analog Beispiel 2.2 durch 1 std. Erwärmen von 1,12 g KOH mit 4,59 g 2-Chlor-3-cyan-6-methyl-5-(pyrid-4-yl)-pyridin in 60 ml Glycerin bei 90-100 °C und zweimaliges Umkristallisieren aus Ethanol unter Zusatz von Aktivkohle.
Ausbeute: 3,2 g (56,1 % der Theorie). Schmp.: 174-5 °C.

Beispiel 3.11

3-Cyan-2-[2-(2-hydroxy-ethoxy)-ethoxy]-6-methyl-5-(pyrid-4-yl)-pyridin

Analog Beispiel 2.2 durch 1,5 std. Erwärmen von 1,3 g KOH mit 4,6 g 2-Chlor-3-cyan-6-methyl-5-(pyrid-4-yl)-pyridin in 50 ml Diethylenglycol unter Rühren bei 90-100 °C und Umkristallisation aus Ethanol und Chloroform/Hexan unter Zusatz von Aktivkohle.
Ausbeute: 3,2 g (53,3 % der Theorie). Schmp.: 119-20 °C.

Beispiel 3.12

2-(2-Acetoxy-ethylamino)-3-cyan-6-methyl-5-(pyrid-4-yl)-pyridin

3,4 g 3-Cyan-2-(2-hydroxy-ethylamino)-6-methyl-5-(pyrid-4-yl)-pyridin werden 1 Std. in 50 ml einer 1:1 Mischung aus Acetanhydrid und Pyridin unter Feuchtigkeitsausschluß unter Rückfluß gekocht. Anschließend wird eingeengt, mit wässrigem $NH_3$ alkalisiert und stehengelassen. Der ausfallende Feststoff wird abgesaugt und aus Ethanol unter Zusatz von Aktivkohle umkristallisiert.
Ausbeute: 3.07 g (77,4 % der Theorie). Schmp.: 146-7 °C.

Beispiel 3.13

3-Cyan-6-methyl-2-(2-propionyloxy-ethoxy)-5-(pyrid-4-yl)-pyridin

4 g 3-Cyan-2-(2-hydroxy-ethoxy)-6-methyl-5-(pyrid-4-yl)-pyridin werden in 40 ml Pyridin mit 4 g Propionsäureanhydrid 0,5 Std. unter Feuchtigkeitsausschluß unter Rückfluß gekocht. Es wird eingeengt, mit Wasser versetzt und stehengelassen. Der ausfallende Feststoff wird abgesaugt und aus Ethanol unter Zusatz von Aktivkohle umkristallisiert.
Ausbeute: 1,6 g (32,8 % der Theorie). Schmp.:98-100 °C.

Beispiel 3.14

3-Cyan-2-(2,3-dibenzoyloxy-propoxy)-6-methyl-5-(pyrid-4-yl)-pyridin

2,28 g 3-Cyan-2-(2,3-dihydroxy-propoxy)-6-methyl-5-(pyrid-4-yl)-pyridin werden in 30 ml Pyridin unter Kühlen und Rühren mit 2 ml Benzoylchlorid versetzt. Es wird 10 Min. bei Raumtemperatur und 30 Min. unter leichtem Erwärmen gerührt, mit Wasser versetzt und mit wässriger NaOH alkalisiert. Die wässrige Phase wird mit Chloroform extrahiert, die Chloroformextrakte werden getrocknet, eingeengt, mit Ethanol und Hexan versetzt und in der Kälte stehengelassen. Das ausfallend Produckt wird abgesaugt und getrocknet.
Ausbeute: 1,1 g (26,9 % der Theorie). Schmp.: 160-2 °C.

Beispiel 3.15

2-(2-Benzoyloxy-propylamino)-3-cyan-6-methyl-5-(pyrid-4-yl)-pyridin-hydrochlorid

Analog Beispiel 3.14 aus 2,5 g 3-Cyan-2-(2-hydroxy-propylamino)-6-methyl-5-(pyrid-4-yl)-pyridin und 2 ml Benzoylchlorid, jedoch Einengen der Chloroformextrakte zur Trockne, Versetzen mit HCl, erneutes Einengen zur Trockne und Umkristallisation aus Ethanol/Diethylether und Trocknen über $P_4O_{10}$ im Vakuum und anschließend bei 110 °C.
Ausbeute: 2,3 g (62 % der Theorie). Schmp.: 130-2 °C.

Beispiel 3.16

2-(2-Amino-ethylamino)-3-cyan-6-methyl-5-(pyrid-4-yl)-pyridin

Analog Beispiel 1.28 aus 2,3 g 2-Chlor-3-cyan-6-methyl-5-(pyrid-4-yl)-pyridin und 2,3 g Ethylendiamin in 50 ml Ethanol, jedoch Einengen zur Trockne, Lösen in 1N HCl, Behandeln mit Aktivkohle, Filtrieren, Fällen mit $NH_3$-Lösung und Umkristallisation aus Ethanol.
Ausbeute: 1,2 g (47,4 % der Theorie). Schmp.:146-9 °C (Zers.).

Beispiel 3.17

3-Cyan-6-methyl-2-piperazino-5-(pyrid-4-yl)-pyridin

3,6 g 2-Chlor-3-cyan-6-methyl-5-(pyrid-4-yl)-pyridin werden mit 3,6 g Piperazin-hexahydrat in 60 ml Ethanol 1 Std. unter Rückfluß gekocht. Es wird über Nacht stehengelassen, filtriert, das Lösungsmittel abdestilliert, mit Wasser versetzt, mit Natronlauge alkalisiert, mit Chloroform extrahiert, die Chloroformextrakte mit Aktivkohle behandelt, getrocknet, das Lösungsmittel abdestilliert und der Rückstand aus Diethylether/Hexan umkristallisiert.
Ausbeute: 2,5 g (57 % der Theorie). Schmp.: 129-30 °C.

Beispiel 3.18

2-(4-Acetyl-piperazino)-3-cyan-6-methyl-5-(pyrid-4-yl)-pyridin

0,5 g 3-Cyan-6-methyl-2-piperazino-5-(pyrid-4-yl)-pyridin werden in 5 ml Pyridin mit 1 ml Acetanhydrid 0,5 Std. unter Feuchtigkeitsausschluß unter Rückfluß gekocht. Es wird eingeengt, mit Wasser versetzt und stehengelassen. Der ausfallende Feststoff wird abgesaugt, getrocknet und anschließend aus Ethanol unter Zusatz von Aktivkohle umkristallisiert.
Ausbeute: 0,4 g (69,5 % der Theorie). Schmp.: 175-80 °C.

Beispiel 4: 2-(Acyloxyalkyl-1-hetero)-3-cyan-5-(pyrid-4-yl)-pyridine

Beispiel 4.1

2-(2-Acetoxy-ethylamino)-3-cyan-5-(pyrid-4-yl)-pyridin

4,8 g 3-Cyan-2-(2-hydroxy-ethylamino)-5-(pyrid-4-yl)-pyridin werden in 40 ml Pyridin mit 3 ml Acetanhydrid 0,5 Std. unter Feuchtigkeitsausschluß unter Rückfluß gekocht. Es wird eingeengt, mit Wasser versetzt und stehengelassen. Der ausfallende Feststoff wird abgesaugt, getrocknet und anschließend aus Ethanol unter Zusatz von Aktivkohle umkristallisiert.
Ausbeute: 4,57 g (80,1 % der Theorie). Schmp.: 141.2 °C.

Beispiel 4.2

2-(3-Acetoxy-propylamino)-3-cyan-5-(pyrid-4-yl)-pyridin

Analog Beispiel 4.1 aus 2,54 g 3-Cyan-2-(3-hydroxy-propylamino)-5-(pyrid-4-yl)-pyridin und 1,5 ml Acetonhydrid in 20 ml Pyridin.
Ausbeute: 2,47 g (79,7 % der Theorie). Schmp.: 137-8 °C.

Beispiel 4.3

2-(2-Acetoxy-ethoxy)-3-cyan-5-(pyrid-4-yl)-pyridin

Analog Beispiel 4.1 aus 4,8 g 3-Cyan-2-(2-hydroxy-ethoxy)-5-(pyrid-4-yl)-pyridin und 3 ml Acetanhydrid in 40 ml Pyridin.
Ausbeute: 4,6 g (81,1 % der Theorie). Schmp.: 111-2 °C.

Beispiel 4.4

2-(3-Acetoxy-propoxy)-3-cyan-5-(pyrid-4-yl)-pyridin

Analog Beispiel 4.1 aus 1,28 g 3-Cyan-2-(3-hydroxy-propoxy)-5-(pyrid-4-yl)-pyridin und 1 ml Acetanhydrid in 10 ml Pyridin.
Ausbeute: 0,7 g (44.3 % der Theorie). Schmp.: 94-6 °C.

Beispiel 4.5

3-Cyan-2-(2-propionyloxy-ethylamino)-5-(pyrid-4-yl)-pyridin

Analog Beispiel 4.1 durch 1,5 std. Kochen von 3,6 g 3-Cyan-2-(2-hydroxy-ethylamino)-5-(pyrid-4-yl)-pyridin mit 3 ml Propionsäureanhydrid in 30 ml Pyridin.
Ausbeute: 4,18 g (94,1 % der Theorie). Schmp. 137-9 °C u. 143-4 °C.

Beispiel 4.6

3-Cyan-2-(3-propionyloxy-propylamino)-5-(pyrid-4-yl)-pyridin

Analog Beispiel 4.1 durch 0,5 std. Kochen von 1,64 g 3-Cyan-2-(3-hydroxy-propylamino)-5-(pyrid-4-yl)-pyridin mit 1,5 ml Propionsäureanhydrid in 12 ml Pyridin.
Ausbeute: 1,59 g (79,5 % der Theorie). Schmp.: 126-7 °C.

Beispiel 4.7

3-Cyan-2-(3-propionyloxy-propoxy)-5-(pyrid-4-yl)-pyridin

Analog Beispiel 4.1 aus 0,241 g 3-Cyan-2-(3-hydroxy-propoxy)-5-(pyrid-4-yl)-pyridin und 0,5 ml, Propionsäureanhydrid in 2 ml Pyridin.
Ausbeute: 0,11 g (35,4 % der Theorie). Schmp.: 59-61 °C.

Beispiel 5.8

2-(2-Benzoyloxy-ethylamino)-3-cyan-5-(pyrid-4-yl)-pyridin

1,2 g 3-Cyan-2-(2-hydroxy-ethylamino)-5-(pyrid-4-yl)-pyridin werden in 10 ml Pyridin unter Rühren mit 2 ml Benzoylchlorid versetzt. Es wird unter leichtem Erwärmen 10 Min. nachgerührt und mit 20 ml Wasser versetzt. Der ausfallende Feststoff wird abgesaugt und mit Wasser, Ethanol und Ether gewaschen.
Ausbeute: 1,5 g (87,2 % der Theorie). Schmp.: 169-71 °C.

Beispiel 4.9

2-(3-Benzoyloxy-propylamino)-3-cyan-5-(pyrid-4-yl)-pyridin

Analog Beispiel 4.8 durch 10 min. Rühren von 1,11 g 3-Cyan-2-(3-hydroxy-propylamino)-5-(pyrid-4-yl)-pyridin mit 1 ml Benzoylchlorid in 10 ml Pyridin.
Ausbeute: 1,45 g (92,9 % der Theorie). Schmp.: 152,5-3,5 °C.

Beispiel 4.10

2-(3-Benzoyloxy-propoxy)-3-cyan-5-(pyrid-4-yl)-pyridin

Analog Beispiel 4.8 durch 10 min. Rühren von 0,48 g 3-Cyan-2-(3-hydroxy-propoxy)-5-(pyrid-4-yl)-pyridin mit 1 ml Benzoylchlorid in 5 ml Pyridin.
Ausbeute: 0.28 g (38,9 % der Theorie). Schmp.: 112-4 °C.

Beispiel 4.11

3-Cyan-2-[2-(2,4-Dimethoxy-benzoyloxy)-ethylamino]-5-(pyrid-4-yl)-pyridin

Durch Versetzen einer Lösung von 0,911 g 2,4-Dimethoxybenzoesäure in 5 ml Pyridin mit 0,6 ml

$SOCl_2$, 5 min. Stehenlassen und Hinzufügen von 1,2 g 3-Cyan-2-(2-hydroxyethyl-amino)-5-(pyrid-4-yl)-pyridin. Nach 15 Min. wird mit 20 ml Wasser und 1 ml 5N $NH_3$ versetzt. Der ausfallende Feststoff wird abgesaugt und aus Ethanol unter Zusatz von Aktivkohle umkristallisiert.
Ausbeute: 0.87 g (43,0 % der Theorie). Schmp.: 155-8 °C. (Gelbfärbung).

Beispiel 4.12

3-Cyan-2-[3-(2,4-dinitro-benzoyloxy)-propylamino]-5-(pyrid-4-yl)-pyridin

Durch Versetzen von 1,27 g 3-Cyan-2-(3-hydroxy-propylamino)-5-(pyrid-4-yl)-pyridin in 10 ml Pyridin mit 1,2 g 2,4-Dinitrobenzoylchlorid, 5 min. Erwärmen, Stehenlassen bei Raumtemperatur, Versetzen mit Wasser, Absaugen und Waschen mit Wasser, Ethanol und Ether.
Ausbeute: 1,45 g (65,7 % der Theorie). Schmp.: 204-7 °C(Zers.).

Beispiel 4.13

3-Cyan-2-(2-nicotinoyloxy-ethylamino)-5-(pyrid-4-yl)-pyridin

3.5 g Nicotinsäure in 20 ml Pyridin werden mit 3,3 ml $SOCl_2$ versetzt. Es wird 5 Min. auf 100 °C erwärmt, abgekühlt, mit 4,8 g 3-Cyan-2-(2-hydroxy-ethylamino)-5-(pyrid-4-yl)-pyridin versetzt, 10 Min. unter Erwärmen auf 50 °C gerührt, abgekühlt und mit ca. 50 ml Wasser versetzt. Der ausfallende Feststoff wird abgesaugt, mit Wasser, Ethanol und Ether gewaschen und aus Methylglykol/Ethanol unter Zusatz von Aktivkohle umkristallisiert.
Ausbeute: 1,7 g (24,6 % der Theorie). Schmp.: 174-5 °C.

Beispiel 4.14

3-Cyan-2-(2-nicotinoyloxy-propylamino)-5-(pyrid-4-yl)-pyridin

Analog Beispiel 4.13 aus 0,369 g Nicotinsäure, 0,33 ml $SOCl_2$, 2 ml Pyridin und 0,509 g 3-Cyan-2-(3-hydroxy-propylamino)-5-(pyrid-4-yl)-pyridin.
Ausbeute: 0,155 g (21,6 % der Theorie). Schmp.: 192-5 °C.

Beispiel 4.15

3-Cyan-2-(3-nicotinoyloxy-propoxy)-5-(pyrid-4-yl)-pyridin

Analog Beispiel 4.13 aus 0.369 g Nicotinsäure, 0,33 ml $SOCl_2$, 2 ml Pyridin und 0,481 g 3-Cyan-2-(3-hydroxy-propoxy)-5-(pyrid-4-yl)-pyridin.
Ausbeute: 0,21 g (29,2 % der Theorie). Schmp.: 160-5 °C. (Zers.).

Beispiel 4.16

2-[2-(5-Butyl-pyridin-2-carbonyloxy)-ethylamino]-3-cyan-5-(pyrid-4-yl)-pyridin

Zu 1,79 g Fusarsäure in 10 ml Pyridin werden langsam unter Rühren bei -20 bis 0 °C 1,6 ml $SOCl_2$ gegeben. Es wird auf Raumtemperatur erwärmt und mit 1,21 g 3-Cyan-2-(2-hydroxy-ethylamino)-5-(pyrid-4-yl)-pyridin in 15 ml Pyridin versetzt. Es wird 1 Std. unter Rückfluß gekocht, abgekühlt, mit Wasser versetzt, zur Trockne eingeengt, in 1N HCl gelöst, mit Aktivkohle versetzt, filtriert und durch Neutralisieren mit 1N $NH_3$ gefällt. Anschließend wird zweimal aus Ethanol/Wasser umkristallisiert.
Ausbeute: 107 mg (5,3 % der Theorie). Schmp.: 165-8 °C.

Beispiel 4.17

2-[Bis-(2-acetoxy-ethyl)-amino]-3-cyan-5-(pyrid-4-yl)-pyridin

Durch 1 std. Kochen von 2,84 g 2-[Bis-(2-hydroxy-ethyl)-amino]-3-cyan-5-(pyrid-4-yl)-pyridin mit 4 ml Acetanhydrid in 20 ml Pyridin, Einengen, Versetzen mit Wasser, Bringen auf pH 7-8, Stehenlassen,

Absaugen und Umkristallisation aus Ethanol/Wasser unter Zusatz von Aktivkohle.
Ausbeute: 2,2 g (59,7 % der Theorie). Schmp.: 90-2 °C.

Beispiel 4.18

2-(2,3-Bisacetoxy-propoxy)-3-cyan-5-(pyrid-4-yl)-pyridin

Durch 0,5 std. Kochen von 5,44 g 3-Cyan-2-(2,3-Dihydroxypropoxy)-5-(pyrid-4-yl)-pyridin mit 4 ml Acetanhydrid in 40 ml Pyridin und Aufarbeiten analog 4.17.
Ausbeute: 5,06 g (71,3 % der Theorie). Schmp.: 76-81 °C.

Beispiel 5: Testungen auf biologische Wirkungen

Beispiel 5.1

Langendorff-Herz in vitro

Für die Versuche wurden männliche Meerschweinchen mit einer, Körpermasse von 300-350 g eingesetzt. An isolierten, mit carbogenbegaster Tyrodelösung von 37 °C durchströmten, frei schlagenden Herzen, wurden unter konstantem Druck die Kontraktionskraft (Inotropie), die Frequenz (Chronotropie) und der Koronardurchfluß unter dem Einfluß der 3-Cyan-2-(heteroalkyl-1-hetero)-5-(pyrid-4-yl)-pyridine der Formel I untersucht. Dazu wurden jeweils 0,1 ml der entsprechend molaren Substanzlösungen als Bolus direkt in den Aortenstumpf appliziert. Die 3-Cyan-2-(heteraalkyl-1-hetera)-5-(pyrid-4-yl)-pyridine zeigten eine teilweise starke und konzentrationsabhängige positiv inotrope und koronardilatatorische Wirkung. Die positiv chronotrope Wirkung war deutlich geringer, ausgeprägt.
Beispielsweise zeigen die Verbindungen der Beispiele 1.2, 1.6, 1.7 und 1.8 eine Erhöhung der Inotropie um 30 % bei Applikation von je 0,1 ml einer 3,0 • $10^{-3}$, 2,2 • $10^{-2}$, 2,0 • $10^{-4}$ bzw. 1,0 • $10^{-3}$ molaren Lösung und eine Erhöhung des Koronardurchflusses um 100 % bei Applikation von je 0,1 ml einer 6,0 • $10^{-3}$, 6,5 • $10^{-2}$, 1,1 • $10^{-2}$ bzw. 1,3 • $10^{-2}$ molaren Lösung. Die Verbindungen der Beispiele 2.2, 2.3 und 2.4 weisen bei Applikation von je 0,1 ml einer $10^{-2}$ molaren Stammlösung in die Aorta eine Erhöhung der Inotropie um 51 %, 38 % und 31 % sowie eine Erhöhung des Koronardurchflusses um 59 %, 99 % bzw. 88 % auf.

Beispiel 5.2

Isolierter, spontanaktiver Vorhof des Meerschweinchens

Für die Versuche wurden männliche Meerschweinchen mit einer Körpermasse von 400-500 g verwendet.
Am isolierten, spontanaktiven Vorhof erfolgte nach einer 60 Min. währenden Adaptationszeit der Organpräparate in 32 °C temperierter carbogen-durchperlter Tyrodelösung die Untersuchung der Beeinflussung von Inotropie und Frequenz unter Einwirkung der 3-Cyan-2-(heteroalkyl-1-hetero)-pyridine der Formel I. Eine Reihe von Verbindungen zeigte eine starke und dosisabhängige positiv inotrope Wirkung.
Bei Verwendung einer Tyrodelösung nach A.A. Alousi et al. [Circ. Res. 45, 666 (1979)] zeigen beispielsweise die Verbindungen von Beispiel 1.2, 1.7, 1.27, 3.3, 3.4, 3.5, 4.1 bzw. 4.3 eine 30%ige Steigerung der Inotropie bei Konzentrationen von 5,6 • $10^{-5}$, 5,4 • $10^{-6}$, 5,0 • $10^{-5}$, 5,8 • $10^{-5}$, 9,1 • $10^{-5}$, 1,6 • $10^{-4}$, 3,5 • $10^{-5}$ und 5,4 • $10^{-5}$ mol/l. Die Verbindung von Beispiel 1.27 zeigt bei einer Konzentration von 5 • $10^{-5}$ mol/l eine Abnahme der Frequenz um 15 %. Die Verbindung von Beispiel 1.8 zeigt bei Verwendung einer Tyrodelösung mit der Zusammensetzung (mmol/l): NaCl 136,9; KCl 2,7; $CaCl_2$ 1,8; $MgCl_2$ 1,0; $NaHCO_3$ 11,9; $NaH_2PO_4$ 0,4; Glucose 16,5 eine 30%ige Steigerung der Inotropie bei einer Konzentration von 1,2 • $10^{-4}$ mol/l.

Beispiel 5.3

Narkotisierter Hund i.v.

Die Untersuchungen wurden an Bastardhunden in Chloralose-Urethan-Narkose bei Prämedikation mit Morphinhydrochlorid unter spontaner Atmung durchgeführt. .

Der linksventrikuläre Druck wurde mittels Tipmanometer und der arterielle Druck wurde über Herzkatheter via A. brachialis gemessen [vgl. K. Femmer et al., Pharmazie 30 642 (1975)]. Unter dem Einfluß der 3-Cyan-2-(heteroalkyl-1-hetero)-pyridine der Formel I wurden eine dosisabhängige beträchtliche. Steigerung der Kontraktionskraft des Herzens sowie teilweise eine signifikante Senkung des peripheren Gesamtwiderstandes beobachtet. So zeigen beispielsweise die Verbindungen von Beispiel 3.2, 4.1, 4.3 und 4.5 eine 50%ige Steigerung des Kontraktilitätsparameters $dp/dt_{max}$ bei einer Dosis von 6,1 • $10^{-6}$, 1,4 • $10^{-6}$, 2,5 • $10^{-6}$ bzw. 2.0 • $10^{-6}$ mol/kg sowie eine 10%ige Senkung des diastolischen Blutdruckes als relevantes Maß einer Abnahme des peripheren Gesamtwiderstandes bei einer Dosis von 2,1 • $10^{-6}$, 1,6 • $10^{-6}$,1,6 • $10^{-6}$ bzw. 1,2 • $10^{-6}$ mol/kg. Die Verbindung von Beispiel 2.1 zeigt bei einer Dosis von 5 mg/kg eine Steigerung von $dp/dt_{max}$ um 126 % Für die Verbindung von Beispiel 2.1 wurde bei einer Dosis von 5 mg/kg eine Senkung des diastolischen Blutdruckes um 37 % gefunden.

Beispiel 5.4

Narkotisiertes Minischwein i.v.

Die Untersuchungen wurden an 6 männlichen Zwergschweinen (Mini-LEWE) mit einem durchschnittlichen Gewicht von 50 kg durchgeführt. Die Tiere wurden unter $N_2O$-$O_2$-Beatmung (3:1) und Muskelrelaxation (d-Tubocurarin) thorakotomiert. Der Blutdruck wurde in der Aorta ascendens mittels Transducer und der Druck im linken Ventrikel (LVP) mit einem Tipmanometer gemessen. Aus dem LVP-Signal wurde über ein Differenzierglied $dp/dt_{max}$ ermittelt. Aus dem EKG wurde mit einem Kardiotachometer die Herzfrequenz zusammen mit anderen Signalen registriert. Das Herz-Zeit-Volumen wurde mit einem elektromagnetischen Flowmeter gemessen und über einen Integrator das Herz-Schlag-Volumen ermittelt.
Einige der 3-Cyan-2-(heteroalkyl-1-hetero)-pyridine der Formel I zeigen an diesem Modell eine starke und dosisabhängige Erhöhung der Kontraktilität des Herzens ($dp/dt_{max}$) sowie teilweise eine signifikante Senkung des totalen peripheren Gesamtwiderstandes. So wurden beispielsweise unter den Verbindungen von Beispiel 1.2 und 1.7 bei einer Dosis von 2,0 • $10^{-6}$ bzw. 5,5 • $10^{-7}$ eine 50%ige Steigerung des Kontraktilitätsparameters $dp/dt_{max}$ sowie bei einer Dosis von 1,1 • $10^{-6}$ bzw. 1,7 • $10^{-6}$ eine 10%ige Senkung des diastolischen Blutdruckes, der neben der beobachteten Steigerung des Herzzeitvolumens eine wesentliche Größe zur Bestimmung des totalen peripheren Gefäßwiderstandes darstellt, gefunden.

Beispiel 5.5

Noradrenalinantagonismus in vitro

Die in Warmblüterphosphattyrode präparierten Samenleiter von Meerschweinchen (ca. 400 g Körpermasse) wurden 60 Min. im Organbad adaptiert. Anschließend wurden die Kontraktionen unter Gabe von Noradrenalin isotonisch registriert. Die 3-Cyan-2-(heteroalkyl-1-hetero)-pyridine der Formel I wurden 1 Min vor den jeweils submaximal reagierenden Noradrenalinkonzentrationen in das Organbad appliziert. Die Verbindungen bewirkten teilweise eine konzentrationsabhängige Hemmung der Noradrenalinkontraktion in vitro, was auf eine periphere vasodilatatorische Wirkkomponente hindeutet.
Für die Verbindungen von Beispiel 1.2, 1.6, 1.7 und 1.8 ergaben sich beispielsweise $ED_{50}$-Werte von 2,2 • $10^{-5}$, 1,6 • $10^{-4}$, 2,5 • $10^{-4}$ sowie 1,3 • $10^{-4}$ mol/l.

Beispiel 5.6

Inhalativer Histaminbronchospasmus

Eine, in einem Ultraschallverdampfer vernebelte 0,1 %ige Histaminlösung induziert an männlichen Meerschweinchen (Körpermasse 200-300 g) innerhalb von 2 bis 3 Min. typische Bronchialkonstriktionen. Durch orale Verabreichung geeigneter Dosen der 3-Cyan-2-(heteroalkyl-1-hetero)-pyridine der Formel I 60 Min. vor der inhalativen Histamingabe wurden die Tiere innerhalb einer festgelegten Expositionszeit vor den durch Histamin verursachten Bronchialkonstriktionen geschützt. Für die Derivate von Beispiel 1.2, 1.6, 1.7 und 1.8 ergeben sich z.B. aus je 10 Tieren pro Dosis $ED_{50}$-Werte von 4,3 • $10^{-4}$, 6,0 • $10^{-4}$, 3,3 • $10^{-4}$ sowie 2,5 • $10^{-4}$ mol/kg, was auf deutliche bronchodilatatorische bzw. antiallergische Wirkungen hinweist.

**Patentansprüche**
**Patentansprüche für folgende Vertragsstaaten : BE, CH, DE, FR, GB, IT, LI, NL, SE**

**1.** Neue 3-Cyan-pyridine der Formel

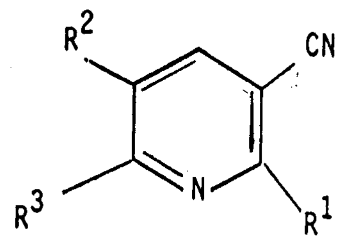

(I)

und ihrer physiologischen verträglichen Säureadditionssalze dadurch gekennzeichnet, daß

$R^1$ Hydroxy-ethylamino, Bis-(2-hydroxyethyl)-amino, Morpholino, 2- oder 3-Hydroxy-propylamino, 1-Hydroxy-methyl-propylamino, 4-Hydroxy-butylamino, 2-Methoxy-ethylamino, 2,2-Dimethoxy-ethylamino, 2-(2-Hydroxy-ethoxy)-ethylamino, 2-Amino-ethylamino, 3-Diethylamino-propylamino, 3-Morpholino-propylamino, Piperazino, 4-Methyl-piperazino, 4-(2-Hydroxyethyl)-piperazino, 2-Acetylamino-ethylamino, 4-Acetyl-piperazino, 4-Propionyl-piperazino, 2-Nicotinoylamino-ethylamino, 2-Methoxy-ethoxy, 2-Hydroxy-ethoxy, 2- oder 3-Hydroxy-propoxy, 2,3-Dihydroxy-propoxy, 2-(2-Hydroxy-ethoxy)-ethoxy, 2-Acetoxy-ethylamino, 3-Acetoxy-propylamino, 2-Acetoxy-ethoxy, 3-Acetoxy-propoxy, Bis-(2-acetoxy-ethyl)-amino, 2,3-Bisacetoxy-propoxy, 2-Propionyloxy-ethylamino, 3-Propionyloxy-propylamino, 3-Propionyloxy-propoxy, 2-Nicotinoyloxy-ethylamino, 3-Nicotinoyloxy-propylamino oder 3-Nicotinoyloxy-propoxy bedeutet, $R^2$ für Pyrid-4-yl und $R^3$ für H oder Methyl stehen.

**2.** Verfahren zur Herstellung von 3-Cyan-pyridinen der Formel I worin $R^1$, $R^2$ und $R^3$ die in Anspruch 1 genannte Bedeutung besitzen, dadurch gekennzeichnet, daß man 3-Cyan-2-halogen-pyridine der Formel II, in der $R^2$ und $R^3$ die oben genannte Bedeutung besitzen,

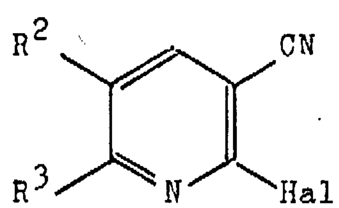

II

a) mit einer Verbindung der Formel

$R^1$ - H III

wenn diese ein Hydroxyalkylamin, Alkoxyalkylamin, Morpholin, Aminoalkylamin, Piperazin oder ein substituiertes Piperazin darstellt, in der $R^1$ die oben genannten Bedeutungen besitzt, zu den 2-Oxyalkylamino- bzw. 2-Aminoalkylamino-Verbindungen

oder

b) mit einer Verbindung der Formel

$R^1$ - H III

wenn diese ein Di- oder Trihydroxyalkan bzw. ein Alkoxyalkanol darstellt, in der $R^1$ die oben genannte Bedeutung besitzt, in Gegenwart von Basen, wie wasserfreie KOH oder NaOH, oder mit den entsprechenden Alkalimetallalkoholaten in dem jeweiligen Alkohol oder in inerten organischen Lösungsmitteln zu den 2-Oxaalkoxy-Verbindungen

oder

c) die nach a) erhaltenen 2-Hydroxyalkylamino- bzw. 2-Aminoalkylamino-Verbindungen der Formel I mit Säureanhydriden oder Säurehalogeniden, vorzugsweise in Gegenwart tertiärer Amine, zu den entsprechenden 2-Acyloxy-alkylamino-, 2-Acylamino-alkylamino- bzw. 2-Acyloxy-alkyloxy-Verbindungen der allgemeinen Formel I

umsetzt und die Verbindungen der Formel I, gegebenenfalls mit anorganischen oder organischen Säuren in die entsprechenden Säureadditionssalze überführt werden.

**3.** Verfahren nach Anspruch 2a, dadurch gekennzeichnet, daß die Umsetzung in organischen Lösungsmitteln wie Alkoholen tertiaren Aminen, Pyridin, Methylglykol, Dioxan, Dimethylformamid, Dimethylsulfoxid, Acetonitril, Gemischen dieser Lösungsmittel sowie den Heteraalkylaminen selbst, erfolgt.

**4.** Pharmazeutisches Mittel, dadurch gekennzeichnet, daß es mindestens eine Verbindung der Formel I gemäß Anspruch 1 und einen physiologisch verträglichen Träger und/oder ein physiologisch verträgliches Verdünnungsmittel enthält.

**5.** Pharmazeutische Zubereitungen dadurch gekennzeichnet, daß sie mindestens eine Verbindung der Formel I gemäß Anspruch 1 und einen geeigneten Trägerstoff enthalten.

**6.** Verwendung der Verhindungen der Formel I gemäß Anspruch 1 zur Herstellung von Arzneimitteln mit kardiotonischen, antiarrhythmischen, vasodilatatorischen, bronchodilatatorischen und/oder antiallergischen Wirkungen.

**7.** Verwendung nach Anspruch 6, dadurch gekennzeichnet, daß Verbindungen der Formel I allein, in Kombination untereinander, mit weiteren kardiotonisch, antiarrhythmisch, vasodilatatorisch, bronchodilatatorisch und/oder antiallergisch wirksamen Verbindungen und/oder mit Trägerstoffen eingesetzt werden.

**8.** Verfahren zur Herstellung von Arzneimitteln nach Anspruch 6 und 7, dadurch gekennzeichnet, daß Verbindungen der Formel I allein eingesetzt oder untereinander oder mit weiteren kardiotonisch, antiarrhythmisch, vasodilatatorisch, bronchodilatatorisch und/oder antiallergisch wirksamen Verbindungen und/oder Trägerstoffen kombiniert werden.

**Patentansprüche für folgenden Vertragsstaat : AT**

**1.** Verfahren zur Herstellung neuer 3-Cyan-pyridine der Formel

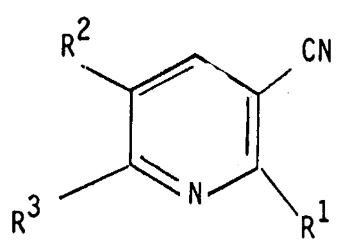

(I)

und ihrer physiologischen verträglichen Säureadditionssalze, dadurch gekennzeichnet, daß in der Formel I $R^1$ Hydroxy-ethylamino, Bis-(2-hydroxyethyl)-amino, Morpholino, 2- oder 3-Hydroxy-propylamino, 1-Hydroxymethyl-propylamino, 4-Hydroxy-butylamino, 2-Methoxy-ethylamino, 2,2-Dimethoxy-ethylamino, 2-(2-Hydroxy-ethoxy)-ethylamino, 2-Amino-ethylamino, 3-Diethylamino-propylamino, 3-Morpholino-propylamino, Piperazino, 4-Methyl-piperazino, 4-(2-Hydroxyethyl)-piperazino, 2-Acetylamino-ethylamino, 4-Acetyl-piperazino, 4-Propionyl-piperazino, 2-Nicotinoylamino-ethylamino, 2-Methoxy-ethoxy, 2-Hydroxy-ethoxy, 2- oder 3-Hydroxy-propoxy, 2,3-Dihydroxy-propoxy, 2-(2-Hydroxy-ethoxy)-ethoxy, 2-Acetoxy-ethylamino, 3-Acetoxy-propylamino, 2-Acetoxy-ethoxy, 3-Acetoxy-propoxy, Bis-(2-acetoxy-ethyl)-amino, 2,3-Bisacetoxy-propoxy, 2-Propionyloxy-ethylamino, 3-Propionyloxy-propylamino, 3-Propionyloxy-propoxy, 2-Nicotinoyloxy-ethylamino, 3-Nicotinoyloxy-propylamino oder 3-

Nicotinoyloxy-propoxy bedeutet, $R^2$ für Pyrid-4-yl und $R^3$ für H oder Methyl stehen.

**2.** Verfahren nach Anspruch 1, dadurch gekennzeichnet, daß $R^1$ gleich Oxaalkylamino für Hydroxy-$C_{1-4}$-alkylamino, Di-(hydroxy-$C_{1-4}$-alkyl)-amino, $C_{1-4}$-Alkoxy-$C_{1-4}$-alkylamino, Di-($C_{1-4}$-alkoxy-$C_{1-4}$-alkyl)-amino, Hydroxy-$C_{1-4}$-alkoxy-$C_{1-4}$-alkylamino oder Morpholino, daß $R^1$ gleich Aminoalkylamino für Di-($C_{1-4}$-alkyl)-amino-$C_{1-4}$-alkylamino, Amino-$C_{1-4}$-alkylamino, Morpholino-$C_{1-4}$-alkylamino oder gegebenenfalls durch $C_{1-4}$-Alkyl oder Hydroxy-$C_{1-4}$-alkyl substituiertes Piperazino, daß $R^1$ gleich Oxaalkyloxy für Hydroxy- oder Dihydroxy-$C_{1-4}$-alkoxy, $C_{1-4}$-Alkoxy-$C_{1-4}$-alkoxy oder Hydroxy-$C_{1-4}$-alkoxy-$C_{1-4}$-alkoxy, daß $R^1$ gleich Acyloxyalkylamino für $C_{2-4}$-Alkanoyloxy-$C_{1-4}$-alkylamino, Di-($C_{2-4}$-alkanoyloxy-$C_{1-4}$-alkyl)-amino oder Pyridylcarbonyloxy-$C_{1-4}$-alkylamino, daß $R^1$ gleich Acylaminoalkylamino für $C_{2-4}$-Alkanoylamino-$C_{1-4}$-alkylamino oder durch $C_{2-4}$-Alkanoyl substituiertes Piperazino oder daß $R^1$ gleich Acyloxyalkyloxy für $C_{2-4}$-Alkanoyloxy-$C_{1-4}$-alkoxy oder Pyridylcarbonyloxy-$C_{1-4}$-alkoxy eingesetzt werden.

**3.** Verfahren zur Herstellung neuer 3-Cyan-pyridine der Formel I, worin $R^1$, $R^2$ und $R^3$ die in den Ansprüchen 1 oder 2 genannte Bedeutung haben, dadurch gekennzeichnet, daß man 3-Cyan-2-halogen-pyridine der Formel II, in der $R^2$ und $R^3$ die in den Ansprüchen 1 bis 2 genannte Bedeutung haben,

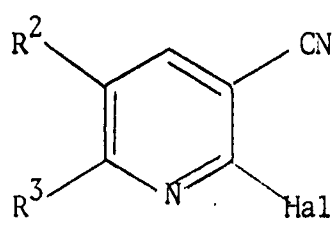

(II)

a) mit Hydroxylalkylaminen, Alkoxyalkylaminen, Morpholin, Aminoalkylaminen bzw. Piperazin oder substituiertem Piperazin zu den 2-Oxaalkylamino- bzw. 2-Aminoalkylamino-Verbindungen
oder
b) mit Di- oder Trihydroxyalkanen bzw. Alkoxyalkanolen in Gegenwart von Basen, wie wasserfreie KOH oder NaOH, oder mit den entsprechenden Alkalimetallalkoholaten in dem jeweiligen Alkohol oder in inerten organischen Lösungsmitteln zu den 2-Oxaalkoxy-Verbindungen
oder
c) die 2-Hydroxyalkylamino- bzw. 2-Aminoalkylamino-Verbindungen aus (a) und die 2-Hydroxyalkyloxy-Verbindungen aus (b) mit Säureanhydriden oder Säurehalogeniden, vorzugsweise in Gegenwart tertiärer Amine, zu den 2-Acyloxy-alkylamino-, 2-Acylamino-alkylamino- bzw. 2-Acyloxyalkyloxy-Verbindungen

umsetzt und die Verbindungen der Formel I gegebenenfalls mit anorganischen oder organischen Säuren in die entsprechenden Säureadditionssalze überführt werden.

**4.** Verfahren nach Anspruch 3, dadurch gekennzeichnet, daß die Umsetzung der Verbindungen der Formel II zu den 2-Oxaalkylamino- bzw. 2-Aminoalkylamino-Verbindungen in organischen Lösungsmitteln, wie Alkoholen, tertiären Aminen, Pyridin, Methylglykol, Dioxan, Dimethylformamid, Dimethylsulfoxid, Acetonitril, Gemischen dieser Lösungsmittel sowie den Heteraalkylaminen selbst, erfolgt.

**5.** Pharmazeutisches Mittel, dadurch gekennzeichnet, daß es mindestens eine Verbindung der Formel I, die gemäß dem Verfahren nach einem der Ansprüche 1 und 2 hergestellt ist, und einen physiologisch verträglichen Träger und/oder ein physiologisch verträgliches Verdünnungsmittel enthält.

**6.** Pharmazeutische Zubereitungen, dadurch gekennzeichnet, daß sie mindestens eine Verbindung der Formel I, die gemäß dem Verfahren nach einem der Ansprüche 1 bis 3 hergestellt ist, und einen geeigneten Trägerstoff enthalten.

**7.** Verwendung der Verbindungen der Formel I, die gemäß dem Verfahren nach einem der Ansprüche 1 und 2 hergestellt sind, zur Herstellung von Arzneimitteln mit kardiotonischen, antiarrhythmischen, vasodilatatorischen, bronchodilatatorischen und/oder antiallergischen Wirkungen.

8. Verwendung nach Anspruch 7, dadurch gekennzeichnet, daß Verbindungen der Formel I allein, in Kombination untereinander, mit weiteren kardiotonisch, antiarrhythmisch, vasodilatatorisch, bronchodilatatorisch und/oder antiallergisch wirksamen Verbindungen und/oder mit Trägerstoffen eingesetzt werden.

9. Verfahren zur Herstellung von Arzneimitteln nach den Ansprüchen 7 und 8, dadurch gekennzeichnet, daß Verbindungen der Formel I allein eingesetzt oder untereinander oder mit weiteren kardiotonisch, antiarrhythmisch, vasodilatatorisch, bronchodilatatorisch und/oder antiallergisch wirksamen Verbindungen und/oder mit Trägerstoffen kombiniert werden.

**Claims**

**Claims for the Contracting States : BE, CH, DE, FR, GB, IT, LI, NL, SE**

1. New 3-cyanopyridines of formula

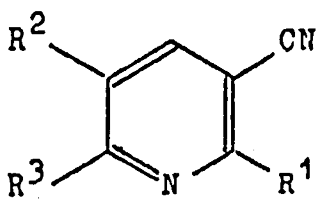

I

and their physiologically well-tolerated acid addition salts, characterized by the fact that $R^1$ represents hydroxyethylamino, bis(2-hydroxyethyl)amino, morpholino, 2- or 3-hydroxypropylamino, 1-hydroxymethylpropylamino, 4-hydroxybutylamino, 2-methoxyethylamino, 2,2-dimethoxyethylamino, 2-(2-hydroxyethoxy)ethylamino, 2-aminoethylamino, 3-diethylaminopropylamino, 3-morpholinopropylamino, piperazino, 4-methylpiperazino, 4-(2-hydroxyethyl)piperazino, 2-acetylaminoethylamino, 4-acetylpiperazino, 4-propionylpiperazino, 2-nicotinoylaminoethylamino, 2-methoxyethoxy, 2-hydroxyethoxy, 2- or 3-hydroxypropoxy, 2,3-dihydroxypropoxy, 2-(2-hydroxyethoxy)ethoxy, 2-acetoxyethylamino, 3-acetoxypropylamino, 2-acetoxyethoxy, 3-acetoxypropoxy, bis(2-acetoxyethyl)amino, 2,3-bisacetoxypropoxy, 2-propionyloxyethylamino, 3-propionyloxypropylamino, 3-propionyloxypropoxy, 2-nicotinoyloxyethylamino, 3-nicotinoyloxypropylamino or 3-nicotinoyloxypropoxy, $R^2$ represents 4-pyridyl, and $R^3$ represents H or methyl.

2. A process for the production of 3-cyanopyridines of Formula I, in which $R^1$, $R^2$ and $R^3$ have the meaning mentioned in Claim 1, characterized by the fact that 3-cynano-2-halopyridines of Formula II, in which $R^2$ and $R^3$ have the above meaning

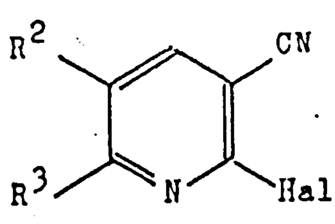

II

are reacted

a) with a compound of Formula

$R^1$ - H III

if this is a hydroxyalkylamine, alkoxyalkylamine, morpholine, aminoalkylamine, piperazine or substituted piperazine, in which $R^1$ has the above meaning, to the 2-oxyalkylamino- or 2-aminoalkylamino compounds

or

b) with a compound of Formula

$R^1$ - H III

if this is a di- or trihydroxyalkane or an alkoxyalkanol, in which $R^1$ has the above meaning, in presence of bases, such as anhydrous KOH or NaOH, or with the corresponding alkali metal alcoholates in the corresponding alcohol or in inert organic solvents to the 2-oxaalkoxy-compounds

or

c) the 2-hydroxyalkylamino- or 2-aminoalkylamino-compounds of Formula I obtained according to a) are reacted with acid anhydrides or acid halides, preferably in presence of tertiary amines, to the corresponding 2-acyloxyalkylamino-, 2-acylaminoalkylamino- or 2-acyloxyalkoxy-compounds of general Formula I

and the compounds of Formula I are optionally converted with inorganic or organic acids to the corresponding acid addition salts.

**3.** A process according to Claim 2a characterized by the fact that the reaction takes place in organic solvents such as alcohols, tertiary amines, pyridine, methoxyethanol, dioxane, dimethylformamide, dimethyl sulphoxide, acetonitrile, mixtures of these solvents and the heteraalkylamines themselves.

**4.** A pharmaceutical agent characterized by the fact that it contains at least one compound of Formula I, according to Claim 1, and a physiologically well-tolerated carrier and/or a physiologically well-tolerated diluent.

**5.** Pharmaceutical preparations characterized by the fact that they contain at least one compound of Formula I, according to Claim 1, and a suitable carrier.

**6.** The use of compounds of Formula I, according to Claim 1, for the production of pharmaceutical preparations with cardiotonic, antiarrhythmic, vasodilator, bronchodilator and/or antiallergic effects.

**7.** The use according to Claim 6, characterized by the fact that compounds of Formula I are used alone or in combination with each other, with other compounds with cardiotonic, antiarrhythmic, vasodilator, bronchodilator and/or antiallergic effects and/or with carriers.

**8.** A process for the production of pharmaceutical preparations according to Claims 6 and 7, characterized by the fact that compounds of Formula 1 are used alone or in combination with each other or with other compounds with cardiotonic, antiarrhythmic, vasodilator, bronchodilator and/or antiallergic effects and/or with carriers.

**Claims for the following Contracting State : AT**

**1.** A process for the production of new 3-cyanopyridines of formula

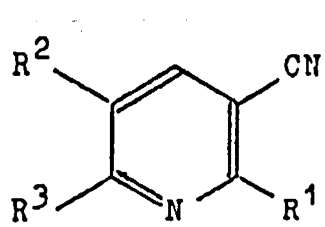

I

and their physiologically well-tolerated acid addition salts, characterized by the fact that in Formula I $R^1$ represents hydroxyethylamino, bis(2-hydroxyethyl)amino, morpholino, 2- or 3-hydroxypropylamino, 1-hydroxymethylpropylamino, 4-hydroxybutylamino, 2-methoxyethylamino, 2,2-dimethoxyethylamino, 2-(2-hydroxyethoxy)ethylamino, 2-aminoethylamino, 3-diethylaminopropylamino, 3-morpholinopropylamino, piperazino, 4-methylpiperazino, 4-(2-hydroxyethyl)piperazino, 2-acetylaminoethylamino, 4-acetylpiperazino, 4-propionylpiperazino, 2-nicotinoylaminoethylamino, 2-methoxyethoxy, 2-hydroxyethoxy, 2- or 3-hydroxypropoxy, 2,3-dihydroxypropoxy, 2-(2-hydroxyethoxy)ethoxy,

2-acetoxyethylamino, 3-acetoxypropylamino, 2-acetoxyethoxy, 3-acetoxypropoxy, bis(2-acetoxyethyl)-amino, 2,3-bisacetoxypropoxy, 2-propionyloxyethylamino, 3-propionyloxypropylamino, 3-propionyloxypropoxy, 2-nicotinoyloxyethylamino, 3-nicotinoyloxypropylamino or 3-nicotinoyloxypropoxy, $R^2$ represents 4-pyridyl,
and $R^3$ represents H or methyl.

**2.** A process according to Claim 1, characterized by the fact that $R^1$ is set equal to oxaalkylamino for hydroxy-$C_{1-4}$-alkylamino, di(hydroxy-$C_{1-4}$-alkyl)amino, $C_{1-4}$-alkoxy-$C_{1-4}$-alkylamino, di($C_{1-4}$-alkoxy-$C_{1-4}$-alkyl)amino, hydroxy-$C_{1-4}$-alkoxy-$C_{1-4}$-alkylamino or morpholino, that $R^1$ is set equal to aminoalkylamino for di($C_{1-4}$-alkyl)amino-$C_{1-4}$-alkylamino, amino-$C_{1-4}$-alkylamino, morpholino-$C_{1-4}$-alkylamino or piperazino, which is optionally substituted by $C_{1-4}$-alkyl or hydroxy-$C_{1-4}$-alkyl, that $R^1$ is set equal to oxaalkoxy for hydroxy- or dihydroxy-$C_{1-4}$-alkoxy, $C_{1-4}$-alkoxy-$C_{1-4}$-alkoxy or hydroxy-$C_{1-4}$-alkoxy-$C_{1-4}$-alkoxy, that $R^1$ is set equal to acyloxyalkylamino for $C_{2-4}$-alkanoyloxy-$C_{1-4}$-alkylamino, di($C_{2-4}$-alkanoyloxy-$C_{1-4}$-alkyl)amino or pyridylcarbonyloxy-$C_{1-4}$-alkylamino, that $R^1$ is set equal to acylaminoalkylamino for $C_{2-4}$-alkanoylamino-$C_{1-4}$-alkylamino or piperazino substituted by $C_{2-4}$-alkanoyl or that $R^1$ is set equal to acyloxyalkoxy for $C_{2-4}$-alkanoyloxy-$C_{1-4}$-alkoxy or pyridylcarbonyloxy-$C_{1-4}$-alkoxy.

**3.** A process for the production of new 3-cyanopyridines of Formula I, in which $R^1$, $R^2$ and $R^3$ have the meaning mentioned in Claims 1 or 2, characterized by the fact that 3-cyano-2-halopyridines of Formula II,

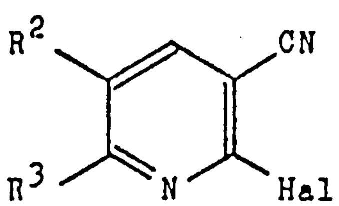

II

in which $R^2$ and $R^3$ have the meaning mentioned in Claims 1 or 2, are reacted

a) with hydroxyalkylamines, alkoxyalkylamines, morpholine, aminoalkylamines or piperazine or substituted piperazine to the 2-oxaalkylamino- or 2-aminoalkylamino-compounds

or

b) with di- or trihydroxyalkanes or alkoxyalkanols in presence of bases, such as anhydrous KOH or NaOH, or with the corresponding alkali metal alcoholates in the corresponding alcohol or in inert organic solvents to the 2-oxaalkoxy-compounds

or

c) the 2-hydroxyalkylamino- or 2-aminoalkylamino-compounds from a) and the 2-hydroxyalkoxy compounds from b) are reacted with acid anhydrides or acid halides, preferably in presence of tertiary amines, to the 2-acyloxyalkylamino-, 2-acylaminoalkylamino-or 2-acyloxyalkoxy-compounds and the compounds of Formula I are optionally converted with inorganic or organic acids to the corresponding acid addition salts.

**4.** A process according to Claim 3, characterized by the fact that the reaction of the compounds of Formula II to the 2-oxaalkylamino- or 2-aminoalkylamino-compounds is carried out in organic solvents, such as alcohols, tertiary amines, pyridine, methoxyethanol, dioxane, dimethylformamide, dimethyl sulphoxide, acetonitrile, mixtures of these solvents or the heteraalkylamines themselves.

**5.** A pharmaceutical agent characterized by the fact that it contains at least one compound of Formula I, which is prepared in accordance with the process of one of Claims 1 and 2, and a physiologically well-tolerated carrier and/or a physiologically well-tolerated diluent.

**6.** Pharmaceutical preparations characterized by the fact that they contain at least one compound of Formula I, which is prepared according to the process of one of Claims 1 to 3, and a suitable carrier.

**7.** The use of compounds of Formula I which are prepared in accordance with the process of one of Claims 1 and 2 for the production of pharmaceutical preparations with cardiotonic, antiarrhythmic,

vasodilator, bronchodilator and/or antiallergic effects.

**8.** The use according to Claim 7, characterized by the fact that compounds of Formula 1 are used alone or in combination with each other or with other compounds with cardiotonic, antiarrhythmic, vasodilator, bronchodilator and/or antiallergic effects and/or with carriers.

**9.** A process for the production of pharmaceutical preparations according to Claims 7 and 8, characterized by the fact that compounds of Formula 1 are used alone or in combination with each other or with other compounds with cardiotonic, antiarrhythmic, vasodilator, bronchodilator and/or antiallergic effects and/or with carriers.

**Revendications**

**Revendications pour les Etats contractants suivants : BE, CH, DE, FR, GB, IT, LI, NL, SE**

**1.** Nouvelles 3-cyanopyridines de formule

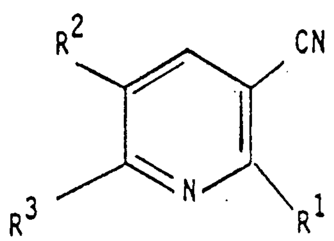

(I)

et leurs sels d'addition d'acide compatibles du point de vue physiologique, caractérisées en ce que $R^1$ représente hydroxy-éthylamino, bis- (2-hydroxyéthyl)-amino, morpholino, 2- ou 3-hydroxy-propylamino, 1-hydroxy-méthyl-propylamino, 4-hydroxy-butylamino, 2-méthoxy-éthylamino, 2,2-diméthoxy-éthylamino, 2-(2-hydroxy-éthoxy)-éthylamino, 2-amino-éthylamino, 3-diéthylamino-propylamino, 3-morpholino-propylamino, pipérazino, 4-méthyl-pipérazino, 4-(2-hydroxyéthyl)-pipérazino, 2-acétylamino-éthylamino, 4-acétyl-pipérazino, 4-propionyl-pipérazino, 2-nicotinoylamino-éthylamino, 2-méthoxy-éthoxy, 2-hydroxy-éthoxy, 2- ou 3-hydroxy-propoxy, 2,3-dihydroxy-propoxy, 2-(2-hydroxyéthoxy)-éthoxy,3-acétoxy-éthylamino, 3-acétoxy-propylamino, 2-acétoxy-éthoxy, 3-acétoxy-propoxy, bis-(2-acétoxy-éthyl)-amino, 2,3-bisacétoxy-propoxy, 2-propionyloxy-éthylamino, 3-propionyloxy-propylamino, 3-propionyloxy-propoxy, 2-nicotinoyloxy-éthylamino, 3-nicotinoyloxy-propylamino ou 3-nicotinoyloxy-propoxy, $R^2$ représente pyridyle-4 et $R^3$ représente H ou méthyle.

**2.** Procédé de préparation de 3-cyanopyridines de formule I dans laquelle $R^1$, $R^2$ et $R^3$ ont la signification indiquée dans la revendication 1, caractérisé en ce que l'on fait réagir des 3-cyano-2-halogénopyridines de formule II dans laquelle $R^2$ et $R^3$ ont la signification indiquée ci-dessus

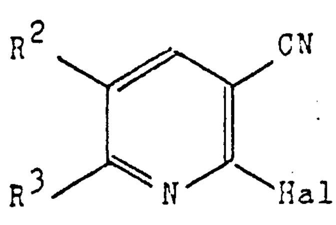

II

a) avec un composé de formule

$R^1$ - H III

lorsque celui-ci représente une hydroxyalkylamine, une alcoxyalkylamine, la morpholine, une aminoalkylamine, la pipérazine ou une pipérazine substituée, dans laquelle $R^1$ possède les significations indiquées ci-dessus, pour former les composés 2-oxyalkylamino ou 2-aminoalkylamino ou bien

b) avec un composé de formule

$R^1$ - H III

lorsque celui-ci représente un di- ou trihydroxyalcane ou un alcoxyalcanol dans lequel $R^1$ possède la signification indiquée ci-dessus, en présence de bases telles que KOH ou NaOH anhydre, ou avec les alcoolates alcalins correspondants pour former l'alcool correspondant, ou dans des solvants organiques inertes pour former les composés 2-oxa-alcoxy ou bien

c) on fait réagir les composés 2-hydroxyalkylamino ou 2-aminoalkylamino de formule I obtenus selon a) avec des anhydrides d'acide ou des halogénures d'acide, de préférence en présence d'amines tertiaires, pour former les composés 2-acyloxy-alkylamino, 2-acylamino-alkylamino ou 2-acyloxy-alkyloxy de formule I correspondants et on convertit éventuellement les composés de formule I en les sels d'addition d'acide correspondants avec des acides inorganiques ou organiques.

**3.** Procédé selon la revendication 2a, caractérisé en ce que la réaction est accomplie dans des solvants organiques tels que les alcools, les amines tertiaires, la pyridine, le méthylglycol, le dioxanne, le diméthylformamide, le diméthylsulfoxyde, l'acétonitrile, des mélanges de ces solvants ainsi que dans les hétéroalkylamines elles-mêmes.

**4.** Agent pharmaceutique caractérisé en ce qu'il contient au moins un composé de formule I selon la revendication 1 et un véhicule compatible du point de vue physiologique et/ou un diluant compatible du point de vue physiologique.

**5.** Préparations pharmaceutiques caractérisées en ce qu'elles contiennent au moins un composé de formule I selon la revendication 1 et un véhicule approprié.

**6.** Utilisation des composés de formule I selon la revendication 1 pour la préparation de médicaments présentant des effets cardiotoniques, antiarythmiques, vasodilatateurs, bronchodilatateurs et/ou antiallergiques.

**7.** Utilisation selon la revendication 6, caractérisée en ce que l'on utilise les composés de formule I seuls, en combinaison entre eux, avec d'autres composés à effet cardiotonique, antiarythmique, vasodilatateur, bronchodilatateur et/ou antiallergique et/ou avec des véhicules.

**8.** Procédé de préparation de médicaments selon les revendications 6 et 7, caractérisé en ce que l'on utilise les composés de formule I seuls ou en combinaison entre eux ou avec d'autres composés à effet cardiotonique, antiarythmique, vasodilatateur, bronchodilatateur et/ou antiallergique et/ou avec des véhicules.

**Revendications pour l'Etat contractant suivant : AT**

**1.** Procédé de préparation de nouvelles 3-cyanopyridines de formule

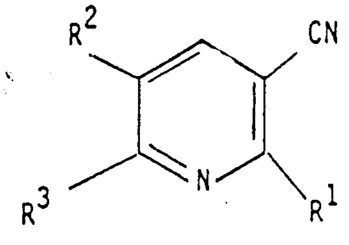

(I)

et de leurs sels d'addition d'acide compatibles du point de vue physiologique, caractérisé en ce que dans la formule I $R^1$ représente hydroxy-éthylamino, bis-(2-hydroxyéthyl)-amino, morpholino, 2- ou 3-hydroxy-propylamino, 1-hydroxy-méthyl-propylamino, 4-hydroxy-butylamino, 2-méthoxy-éthylamino, 2,2-diméthoxy-éthylamino, 2-(2-hydroxy-éthoxy)-éthylamino, 2-amino-éthylamino, 3-diéthylamino-propylamino, 3-morpholino-propylamino, pipérazino, 4-méthyl-pipérazino, 4-(2-hydroxyéthyl)-pipérazino, 2-acétylamino-éthylamino, 4-acétyl-pipérazino, 4-propionyl-pipérazino, 2-nicotinoylamino-éthylamino, 2-méthoxy-éthoxy, 2-hydroxy-éthoxy, 2- ou 3-hydroxy-propoxy, 2,3-dihydroxy-propoxy, 2-(2-hydroxy-éthoxy)-éthoxy, 3-acétoxy-éthylamino, 3-acétoxy-propylamino, 2-acétoxy-éthoxy, 3-acétoxy-propoxy, bis-(2-acétoxy-éthyl)-amino, 2,3-bisacétoxy-propoxy, 2-propionyloxy-éthylamino, 3-propionyloxy-propylamino, 3-propionyloxy-propoxy, 2-nicotinoyloxy-éthylamino, 3-nicotinoyloxy-propylamino ou 3-nicotinoyloxy-propoxy, $R^2$ représente pyridyle-4 et $R^3$ représente H ou méthyle.

**2.** Procédé selon la revendication 1, caractérisé en ce que l'on utilise $R^1$ égal à oxaalkylamino pour hydroxy-alkyle en $C_{1-4}$-amino, di-(hydroxy-alkyle en $C_{1-4}$)-amino, alcoxy en $C_{1-4}$-alkyle en $C_{1-4}$-amino, di-(alcoxy en $C_{1-4}$-alkyle en $C_{1-4}$)-amino, hydroxy-alcoxy en $C_{1-4}$-alkyle en $C_{1-4}$-amino ou morpholino, en ce que l'on utilise $R^1$ égal à aminoalkylamino pour di-(alkyle en $C_{1-4}$)-amino-alkyle en $C_{1-4}$-amino, amino-alkyle en $C_{1-4}$-amino, morpholino-alkyle en $C_{1-4}$-amino ou pipérazino éventuellement substitué par alkyle en $C_{1-4}$ ou hydroxy-alkyle en $C_{1-4}$, en ce que l'on utilise $R^1$ égal à oxaalkyloxy pour hydroxy- ou dihydroxy-alcoxy en $C_{1-4}$, alcoxy en $C_{1-4}$-alcoxy en $C_{1-4}$ ou hydroxy-alcoxy en $C_{1-4}$-alcoxy en $C_{1-4}$, en ce que l'on utilise $R^1$ égal à acyloxyalkylamino pour alcanoyloxy en $C_{1-4}$-alkyle en $C_{1-4}$-amino, di-(alcanoyloxy en $C_{2-4}$-alkyle en $C_{1-4}$)-amino ou pyridylcarbonyloxy-alkyle en $C_{1-4}$-amino, en ce que l'on utilise $R^1$ égal à acylaminoalkylamino pour alcanoyle en $C_{2-4}$-amino-alkyle en $C_{1-4}$-amino ou pipérazino substitué par alcanoyle en $C_{2-4}$ ou en ce que l'on utilise $R^1$ égal à acyloxyalkyloxy pour alcanoyloxy en $C_{1-4}$-alcoxy en $C_{1-4}$ ou pyridylcarbonyloxy-alcoxy en $C_{1-4}$.

**3.** Procédé de préparation de nouvelles 3-cyanopyridines de formule I dans laquelle $R^1$, $R^2$ et $R^3$ ont la signification indiquée dans les revendications 1 ou 2, caractérisé en ce que l'on fait réagir des 3-cyano-2-halogénopyridines de formule II dans laquelle $R^2$ et $R^3$ ont la signification indiquée dans les revendications 1 et 2

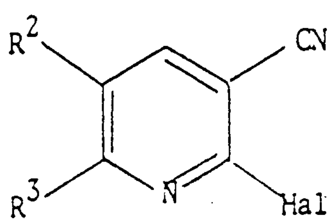

(II)

a) avec des hydroxylalkylamines, des alcoxyalkylamines, la morpholine, des aminoalkylamines ou la pipérazine ou une pipérazine substituée pour former les composés 2-oxaalkylamino ou 2-aminoalkylamino ou bien

b) avec des di- ou trihydroxyalcanes ou des alcoxyalcanols en présence de bases telles que KOH ou NaOH anhydre, ou avec les alcoolates alcalins correspondants pour former l'alcool correspondant, ou dans des solvants organiques inertes pour former les composés 2-oxaalcoxy ou bien

c) on fait réagir les composés 2-hydroxyalkylamino ou 2-aminoalkylamino obtenus selon a) et les composés 2-hydroxyalkyloxy obtenus selon b) avec des anhydrides d'acide ou des halogénures d'acide, de préférence en présence d'amines tertiaires, pour former les composés 2-acyloxy-alkylamino, 2-acylamino-alkylamino ou 2-acyloxy-alkyloxy

et on convertit éventuellement les composés de formule I en les sels d'addition d'acide correspondants avec des acides inorganiques ou organiques.

**4.** Procédé selon la revendication 3, caractérisé en ce que la réaction des composés de formule II pour former les composés 2-oxaalkylamino ou 2-aminoalkylamino est accomplie dans des solvants organiques tels que les alcools, les amines tertiaires, la pyridine, le méthylglycol, le dioxanne, le diméthylformamide, le diméthylsulfoxyde, l'acétonitrile, des mélanges de ces solvants ainsi que dans les hétéroalkylamines elles-mêmes.

**5.** Agent pharmaceutique caractérisé en ce qu'il contient au moins un composé de formule I qui est préparé par le procédé selon l'une des revendications 1 et 2 et un véhicule compatible du point de vue physiologique et/ou un diluant compatible du point de vue physiologique.

**6.** Préparations pharmaceutiques caractérisées en ce qu'elles contiennent au moins un composé de

formule I qui est préparé par le procédé selon l'une des revendications 1 à 3 et un véhicule approprié.

**7.** Utilisation des composés de formule I qui sont préparés par le procédé selon l'une des revendications 1 et 2 pour la préparation de médicaments présentant des effets cardiotoniques, antiarythmiques, vasodilatateurs, bronchodilatateurs et/ou antiallergiques.

**8.** Utilisation selon la revendication 7, caractérisée en ce que l'on utilise les composés de formule I seuls, en combinaison entre eux, avec d'autres composés à effet cardiotonique, antiarythmique, vasodilatateur, bronchodilatateur et/ou antiallergique et/ou avec des véhicules.

**9.** Procédé de préparation de médicaments selon les revendications 7 et 8, caractérisé en ce que l'on utilise les composés de formule I seuls ou en combinaison entre eux ou avec d'autres composés à effet cardiotonique, antiarythmique, vasodilatateur, bronchodilatateur et/ou antiallergique et/ou avec des véhicules.